# EUROPEAN PATENT SPECIFICATION

(11) **EP 3 545 103 B1**
(45) Date of publication and mention of the grant of the patent: **02.03.2022**
(21) Application number: 17811511.9
(22) Date of filing: 27.11.2017
(51) Int. Cl.: C12Q 1/6883

(54) **METHOD AND BIOMARKERS FOR IN VITRO DIAGNOSIS OF MENTAL DISORDERS**
VERFAHREN UND BIOMARKER ZUR IN-VITRO-DIAGNOSE VON MENTALEN STÖRUNGEN
PROCÉDÉ ET BIOMARQUEURS POUR DIAGNOSTIC IN VITRO DE TROUBLES MENTAUX

(30) Priority: 28.11.2016 EP 16200925
(43) Date of publication of application: 02.10.2019
(73) Proprietor: Korth, Carsten, 40219 Düsseldorf (DE)
(72) Inventor: TROSSBACH, Svenja, 40223 Düsseldorf (DE); HECHER, Laura, 20251 Hamburg (DE); KORTH, Carsten, 40219 Düsseldorf (DE)
(74) Representative: Remus, Alvaro Johannes
(86) International application number: PCT/EP2017/080504
(87) International publication number: WO 2018/096141

(56) References cited:
- WO-A1-03/013551
- WO-A1-2010/097631
- WO-A2-2008/048639
- WO-A2-2009/102748
- HETTEMA JOHN M: "Genetic association between RGS1 and internalizing disorders", PSYCHIATRIC GENETICS, RAPID COMMUNICATIONS OF OXFORD LTD, XX, vol. 23, no. 2, 1 April 2013 (2013-04-01), pages 56-60, XP009193991, ISSN: 0955-8829, DOI: 10.1097/YPG.0B013E32835D7048
- S V TROSSBACH ET AL: "Misassembly of full-length Disrupted-in-Schizophrenia 1 protein is linked to altered dopamine homeostasis and behavioral deficits", MOLECULAR PSYCHIATRY, vol. 21, no. 11, 12 January 2016 (2016-01-12), pages 1561-1572, XP055441893, GB ISSN: 1359-4184, DOI: 10.1038/mp.2015.194

## Description

### Field of the invention

The invention relates to a method for *in vitro* diagnosis of the presence of a mental disorder in a human individual or the predisposition of the human individual to the mental disorder.

### Background of the invention

Mental disorders such as schizophrenia or depression are so far solely diagnosed by a clinical approach through an interview based on the patient's self- reported experiences, behaviour reported by relatives or friends, and a mental status exam. There is no reliable and useful objective laboratory test for mental disorders, for example, based on a biological cause. That unmated clinical diagnosis is unsatisfactory by the reason that a small part of subjectivity remains and that the whole test is addicted to an observer. Another issue is the point that the heterogeneous disease course suggests different biological causes. In one case an illness runs with one single episode and has no residuum, in another case multiple episodes with no or minimum residuum are the result, such as a case which is characterised by a residuum after a first episode falling into relapse without the return to normality or a case with progressing residuum after each episode of the disorder without a return to normality. The various ways of a mental disorder makes it harder to correctly diagnose the illness by mere clinical diagnosis only with the aim of finding the right way to treat the patients. However, patients and their affiliated persons such as the pharmaceutical companies are interested on an objective and independent method to diagnose mental disorders like schizophrenia or depression.

It is assumed that there are several biological causes for mental disorders such as schizophrenia or depression, which, however, all converge in a common behavioural pathway which then can give the illusion of a homogeneity of an underlying biology. For example, typical biological symptoms of a schizophrenia patient are the increased striatal dopamine levels. Another possible symptom are neuroanatomical abnormalities such as enlarged ventricles, aberrant interneuron positioning, or biochemical abnormalities such as abnormal proteostasis of some key proteins. Based on this knowledge an objective method to diagnose a mental disorder like schizophrenia should be discovered.

For example, disrupted in schizophrenia 1 (DISC1) is a protein that has been shown to participate in the regulation of cell proliferation, differentiation, migration, neuronal axon and dendrite outgrowth, mitochondrial transport, fission and/or fusion, and neurotransmitter functions at the synapse. Several studies have shown that unregulated expression may predispose animals to behavioural abnormalities or human individuals to the development of schizophrenia, clinical depression, bipolar disorder, and other psychiatric conditions. It has been hypothesized that unbalanced proteostasis in neurons may lead to DISC1 protein aggregates in a subset of patients with schizophrenia or other chronic mental disorders, and several findings justify classification of DISC1-dependent brain disorders as protein conformational disorders which we have tentatively termed DISC1 opathies (Korth, 2012). That is, disturbed proteostasis and protein aggregation can be considered as a mechanism of mental disorders.

### Prior art

Dean (2011) outlines the evolving notion of biomarkers for diagnosing mental disorders, especially schizophrenia, and discloses outcomes from a variety of biomarkers discovery strategies. In particular, the impact of high-throughput screening technologies on biomarker discovery is highlighted and how new or improved technologies may allow the discovery of either diagnostic biomarkers for schizophrenia or biomarkers that will be useful in determining appropriate treatments for people with the disorder. It is suggested that biomarkers can be identified and that these biomarkers will be useful in diagnosing and treating people with schizophrenia. However, although Dean suggests some proteins as potential biomarkers for schizophrenia and bipolar disorders, the author admits that those biomarkers still have to be validated and the development of clinically useful markers may still take a long time.

Chan et al. (2015) describe the development of a serum biomarker test for the identification of individuals at risk of developing schizophrenia based on multiplex immunoassay profiling analysis. A meta-analysis of independent cohorts of first-onset drug-naive schizophrenia patients and controls was conducted. Using least absolute shrinkage and selection operator regression, an optimal panel of biomarkers that best discriminated patients and controls was identified. This biomarker panel was verified using two independent validation cohorts and its predictive performance for identifying patients before onset of psychosis was tested using two cohorts of pre-onset or at-risk individuals. These findings are alleged to represent the first successful step towards a test that could address the clinical need for early intervention in psychiatry. However, this study does not consider underlying biological heterogeneity of schizophrenia as the identification of biomarkers starts in patient cohorts defined by clinical diagnosis.

WO 2013/186562 A1 discloses a biomarker set for diagnosing disorders like depression, anxiety disorder or other psychotic disorders consisting of eleven markers. It is suggested to use one or more analytes selected from Interleukin 10 (IL-10), Interleukin 18 (IL-18), Interleukin 2 (IL-2), Interleukin 8 (IL-8), Monocyte Chemotactic Protein 1 (MCP- 1), Macrophage Inflammatory Protein 1 alpha (MIP-Iα), Macrophage Inflammatory Protein 1 beta (MIP-Iβ), Matrix Metalloproteinase 2 (MMP-2), Tumor Necrosis Factor beta (TNF-β), Interleukin 4 (IL-4), and Interferon gamma (IFN-γ) as a biomarker for such diseases, or predisposition thereto. WO 2013/186562 A1 further discloses a method of diagnosing depression, anxiety disorder or other psychotic disorders in an individual, wherein the amounts of said analyte biomarkers in a biological sample obtained from the individual are quantified and then compared with the amounts present in a normal control sample from a normal subject, such that a difference in the level of the analyte biomarkers in the biological sample is indicative of the disorder, or predisposition thereto. The data provided show that the analytes may be statistically significant biomarkers for the diagnosis of depression and anxiety disorder. In particular, innate immune responsiveness is increased in persons with depressive and anxiety disorders, indicating a possible genetic vulnerability for depression or anxiety.

From WO 2010/097631 A1 it is known to use IL-17, IgA, Cortisol (CORT), Apolipoprotein Al, IL-6, Complement 3 (C3), Factor VII, Serum Amyloid P (SAP or APCS), Beta 2 Microglobulin, ICAM-I, IL-I beta, TNF alpha, MIF, Angiotensinogen, NrCAM (Neuronal cell adhesion molecule), CD40, Cancer Antigen 125 (CA125), HCC 4 (CCL6; SCYA6), Eotaxin 3 (CCL26 or SCYA26), VEGF, Haptoglobin (HP), IL-I alpha, Apolipoprotein H (Beta-2 Glycoprotein) and TIMP 1 as a specific panel of analyte biomarkers for major depressive disorder, or predisposition thereto. This panel of biomarkers can be used in a method of diagnosing or monitoring major depressive disorder, or predisposition thereto, wherein said analyte biomarkers are detected and/or quantified in a sample from a test subject. The levels of these analyte biomarkers are found to be increased in patients with major depressive disorder.

US 2011/0136738 A1 discloses a method for identifying gene targets which are associated with schizophrenia or schizophrenia-like symptoms. Animal models of schizophrenia were utilized, and initiated, and from tissue obtained from at least one of those animals, transcriptional regulation was assessed over time, relative to the onset of the schizophrenia model. It is suggested to measure gene expression from animals at time points after, and, optionally, before the initiation of the model and to compare the gene expression, whether from before or after the initiation of the model, or both, to gene expression at one or more time points from control animals, which are not subject to a schizophrenia model. Transcripts can then be detected which are dysregulated in tissue from animals that are a model of schizophrenia. Any change in gene expression observed in the schizophrenia model, whether relative to other time points in the same model, relative to another schizophrenia model, or relative to the same time point or time points in control animals can be informative with respect to gene targets for schizophrenia or the symptoms of schizophrenia. However, although several genes have been identified, the dysregulation of which seems to be indicative of the presence of schizophrenia or the symptoms thereof, this document does neither provide any clinically suitable biomarkers nor any validated test for diagnosing schizophrenia.

WO 2003/013551 A1 discloses methods and compositions for the diagnosis, treatment and prognosis of G-protein coupled receptor (GPCR)-related disorders through inhibition of regulators of G-protein signaling (RGS) proteins. The methods relate to screening and assessing test compounds useful in the intervention and prevention of GPCR-related disorders including neuropsychiatric and cardiopulmonary disorders. Further disclosed are methods to identify inhibitors for RGS expression or activity which are useful in the modulation of GPCR signaling pathways.

Accordingly, there is a need to identify biological causes involved in mental disorders such as schizophrenia, as well as for methods that can detect such causes so they can be utilized in screening therapeutics, in diagnosing mental disorders, and in developing treatments for individuals with mental disorders. These biological causes may be manifold and consist in genes, protein pathology, or others. There is also a need for new biomarkers and methods for diagnosing mental disorders or detecting susceptibility to mental disorders, and for preventing or following up development of such disorders.

Moreover, patients and their relatives want an "objective" diagnosis rather than an oral verdict and pharmaceutical companies want an "objective" test to base 100-million EU clinical trials thereon, rather than a clinical diagnosis. However, although some single biomarkers or rather large biomarker sets for diagnosing mental disorders are already known, there is still no reliable and precise test which would be suitable to replace or even support the common clinical approach. In contrast, the current issue with the known biomarker tests is that they involved only a few and unspecific or too many biomarkers so that they get either inaccurate or too extensive and thus are not reliable and deliver aberrant diagnoses. Yet another problem in current biomarker identification is that this identification starts in patient cohorts defined by clinical diagnosis and not considering underlying biological heterogeneity. As a consequence of this procedure, any possible specific effects in patient subsets are diluted out.

### Summary of the invention

It is the objective of the invention as defined by the appended claims to provide a method for *in vitro* diagnosis of the presence of a mental disorder in a human individual or the predisposition of the human individual to the mental disorder, as well as at least one marker protein, nucleic acid molecule, or combination of marker proteins or nucleic acid molecules for use in such method, which ensure an accurate and reliable diagnosis of mental disorders.

This objective is met by a method for *in vitro* diagnosis of the presence of a mental disorder in a human individual or the predisposition of the human individual to the mental disorder, wherein the mental disorder is associated with a dysfunctional DISC1 protein pathway or disturbed dopamine homeostasis, and wherein said method comprises:
a) measuring in a sample of a body tissue or fluid from the individual the expression levels of at least two marker genes, each of which coding for at least one marker protein, wherein said marker genes are selected from the group consisting of human *NKG7, RGS1, CCL4, IFNG, IL12RB2, IL13RA1, KMO, FPR2, SLC27A2,* and C3;
b) comparing the measured expression levels to predetermined threshold values representing the expression levels of said marker genes in a healthy population; and
c) based on the comparison, determining whether the individual has the mental disorder or a predisposition to the mental disorder, wherein the measured expression levels are indicative to the mental disorder or disposition if the measured expression levels of said marker genes exceed, reach or fall below the predetermined threshold value.

In the method according to the invention the measured expression levels of at least two marker genes are combined in order to determine whether the individual has the mental disorder or a predisposition to the mental disorder in step c). Combining two or more markers significantly increases specificity of the method according to the invention. In some cases, sensitivity of the method may be decreased at the same time. However, in the method according to the invention specificity is more important than sensitivity since the method is provided for a sub-group of patients only and thus low sensitivity relating to the all-comprising clinical diagnosis is expected and can therefore be neglected.

The threshold values are predetermined by empirically determining a reference (standard) expression level for each marker gene, i.e. the average expression level of the marker gene in a healthy population. The measured expression levels of the marker genes in the body tissue or fluid sample of the individual (patient) are each compared to the respective predetermined threshold value. If the measured expression levels of said marker genes exceed, reach or fall below the predetermined threshold values (the direction of the change depends on whether the respective aberrant expression level is higher or lower than the respective normal expression level), it is indicated that the individual has the mental disorder or at least a predisposition thereto. That is, altered expression levels of the marker genes in the body tissue or fluid sample relative to the expression levels of the same marker genes in the normal control (reference or standard expression levels of a healthy population) are indicative of the presence of the mental disorder, or predisposition thereto.

According to the invention the method is focused on a subset of so far purely clinically defined mental disorders that are associated with a dysfunctional DISC1 protein pathway or disturbed dopamine homeostasis. That is, the method according to the invention is useful for a subset of patients that are afflicted with mental disorders caused by disturbed homeostasis of DISC1 protein / pathway or dopamine homeostasis. For example, unregulated expression, degradation or altered protein structure of DISC1 may predispose individuals to the development of schizophrenia, recurrent depression, bipolar disorder, and other psychiatric conditions. So-called DISC1opathies seem to be caused by unbalanced proteostasis in neurons leading to DISC1 protein aggregates so that DISC1-dependent brain disorders may be classified as protein conformational disorders. Accordingly, protein aggregation can be deemed as a biological phenotype for sporadic chronical mental disorders for a subgroup of patients, for example schizophrenia or depression patients.

Sporadic disorders are understood to represent disease entities where no clear and unambiguous genetic cause can be identified.

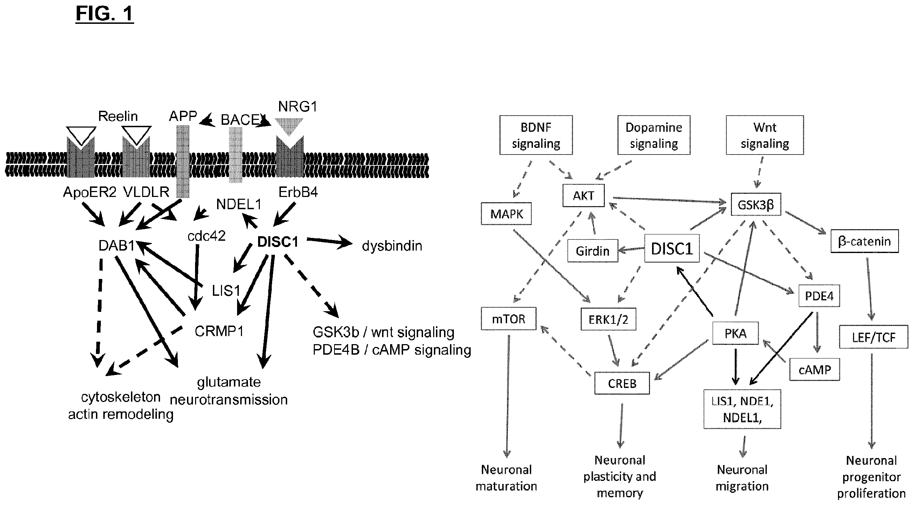

The extended DISC1 pathway includes many other genes, also involved in either mental illness or neurodegenerative disease **(****Figure 1**; Hennah & Porteous 2009; Soares et al. 2011; Korth 2009 und 2012). The marker genes listed in **Table 1** (and their human equivalents), in particular *NKG7, RGS1, CCL4, IFNG, IL12RB2, IL13RA1, KMO, FPR2, SLC27A2* and C3, belong to a network of genes directly or indirectly regulated by DISC1, DISC1-associated proteins, or the DISC1 pathway and are therefore potentially useful as biomarkers for diagnosing mental disorders that are associated with a dysfunctional DISC1 protein pathway.

Dopamine also plays a critical role in the genesis of psychosis, the acute symptom of schizophrenia. It is hypothesized that a framework exists which links risk factors, including pregnancy and obstetric complications, stress and trauma, drug use, and genes, to increased presynaptic striatal dopaminergic function. This hypothesis explains how a complex array of pathological, positron emission tomography, magnetic resonance imaging, and other findings, such as frontotemporal structural and functional abnormalities and cognitive impairments, may converge neurochemically to cause psychosis through aberrant salience and lead to a diagnosis of schizophrenia (Howes & Kapur 2009). Moreover, on a molecular mechanistic level, DISC1 missassembly seems to modulate dopamine homeostasis. DISC1opathies thus define a biology-based category of human mental illnesses with involvement of the dopamine system that can be modeled in animals, i.e. in a transgenic animal (e.g., tgDISC1 rat). Accordingly, marker proteins involved in dopamine homeostasis may also be potentially useful as biomarkers for diagnosing mental disorders that are associated with a dysfunctional DISC1 protein pathway.

Known tests for diagnosing schizophrenia start biomarker discovery with the clinical diagnosis of the disease, which is doomed to fail because of underlying clinical heterogeneity that dilutes any possible significant biomarkers for subsets of clinically defined mental disorders, and known array tests generated by proteomics use surrogate markers rather than markers based on biological causes, which results in low plausibility. The method according to the invention is based on a biological definition (e.g., "DISC1opathy") and a biological (animal) model thereof. Accordingly, the marker genes used therein (*NKG7, RGS1, CCL4, IFNG, IL12RB2, IL13RA1, KMO, FPR2, SLC27A2,* and C3) belong to a network of genes directly or indirectly regulated by DISC1-associated proteins. This advantageous approach provides high plausibility as it is based on a subgroup of patients with a defined biological cause and thus avoids to be affected by clinical and biological heterogeneity of the various mental disorders. Using the human equivalents of the marker genes selected from the group consisting of *NKG7, RGS1, CCL4, IFNG, IL12RB2, IL13RA1, KMO, FPR2, SLC27A2,* and C3, the method according to the invention therefore ensures an accurate and reliable diagnosis of mental disorders that are associated with a dysfunctional DISC1 protein pathway or disturbed dopamine homeostasis. The marker genes and proteins used herein also comprise processed proteins or splice variants, i.e. molecules derived from the original gene or protein indicated.

In an advantageous embodiment of the invention a first marker gene is *RGS1* and at least one second marker gene is selected from the group consisting of human *NKG7, CCL4, IFNG, IL12RB2, IL13RA1, KMO, FPR2, SLC27A2,* and C3. These marker genes or the transcripts or related marker proteins thereof, alone or in various combinations, are particularly advantageous for diagnosing mental disorders associated with a dysfunctional DISC1 protein pathway or disturbed dopamine homeostasis. Preferably, *RGS1* is combined with *CCL4* and/or *NKG7.* Especially *RGS1* is correlated to cognitive endophenotypes of patients and also clearly decreased in patients. On the other hand, the NK cell markers CCL4 and NKG7 have by themselves already a high diagnostic potential, however they are not expressed in the brain. The decreased NK cell genes *NKG7* and *CCL4* have no overlap with *RGS1* in terms of co-regulation. Therefore, *RGS1* in conjunction with an NK cell marker is advantageous to diagnose the subset with credibility, plausibility and specificity. No single marker is likely able to do this because one is not expressed in the brain, the other not specific enough.

In another advantageous embodiment of the invention at least one additional marker gene is selected from the human equivalents of the genes listed in Table 1. Using additional marker may further increase sensitivity and/or sensitivity of the method according to the invention. The marker genes and proteins indicated in Table 1 also comprise processed proteins or splice variants, i.e. molecules derived from the original gene or protein indicated.

A lower expression level of the marker genes in the body tissue or fluid sample relative to the respective expression levels in the normal controls (reference or standard expression levels of a healthy population) is indicative of the presence of the mental disorder, or predisposition thereto. Surprisingly, the marker genes selected from the group consisting of human *NKG7, RGS1, CCL4, IFNG, IL12RB2, IL13RA1, KMO, FPR2, SLC27A2,* and *C3* are all downregulated, i.e. their transcript level is decreased relative to the reference (standard) level of a healthy population. In contrast, prior art assumes increased marker levels, particularly, with markers for pro-inflammatory cytokines. The decreased expression levels of the marker genes according to the invention therefore seem to represent a subset of cases with decreased inflammatory markers. Surprisingly, this is not in contradiction to previous findings because the decrease of these markers in a subset is diluted out and overcompensated by an increase of the same markers in the other subsets such that when the all-comprising clinical diagnosis is used the markers appear slightly increased.

In another advantageous embodiment of the invention, the body tissue or fluid is selected from whole blood, blood plasma, blood serum, cerebrospinal fluid, urine, saliva, biopsy material, and/or isolated cells and their ex *vivo* derivatives. For example, the body fluid can also be modelled by taking cells from a patient or human individual and reprogramming those to various cell types (induced pluripotent stem (iPS) cells and differentiation of those).

In another advantageous embodiment of the invention the expression levels of the marker genes (transcript levels) may be measured by quantitative reverse transcription Polymerase Chain Reaction (qRT-PCR), quantitative real-time PCR, or any other method suitable to determine the amount of transcript (mRNA or cDNA) within the body fluid. Alternatively, the expression levels of the marker proteins (protein levels) can be measured by any high-affinity binding assay, for example, an immune-/antibody-based assay such as an Enzyme Linked Immunosorbent Assay (ELISA). The high-affinity binding assay is not limited to antibodies but can also consist of peptides, small nucleic acids called aptamers, even small organic molecules, or others. It is also conceivable to combine both techniques to what is called immuno PCR.

For example, the expression levels may be determined using naturally occurring or chemically synthesized compounds capable of specifically binding to the respective marker protein or the transcripts (mRNA) of the marker genes. Such compounds may be selected from the group comprising a peptide, an antibody or a fragment thereof, an aptamer (peptide or oligonucleotide), and an oligonucleotide. The compound may be labelled with a detectable label, such as a luminescent, fluorescent or radioactive group. Alternatively or additionally, the compound may be labelled with an affinity tag, e.g., a biotin, avidin, streptavidin or His (e.g. hexa-His) tag. For high-throughput applications an array comprising the compound may be used, e.g., a microarray in the form of a biochip.

Most notably, and especially if a high-throughput assay shall be established, the expression level of the marker protein is measured by means of a microarray analysis (biochip technology).

The method according to the invention may be, for example, performed by means of a kit for diagnosing the presence of a mental disorder in a human individual or the predisposition of the human individual to the mental disorder in *vitro,* wherein the mental disorder is associated with a dysfunctional DISC1 protein pathway or disturbed dopamine homeostasis, the kit comprising:
a) a set of oligonucleotide primers which are suitable to initiate amplification of the transcripts of at least two marker genes, each of which coding for at least one marker protein, in a Polymerase Chain Reaction and/or microarray, wherein said marker genes are selected from the group consisting of human *NKG7, RGS1, CCL4, IFNG, IL12RB2, IL13RA1, KMO, FPR2, SLC27A2,* and C3, and/or
   at least two first antibodies or molecules, each of which specifically binding to a marker protein in a body tissue or fluid from the individual, wherein the marker proteins are derived from marker genes selected from the group consisting of human *NKG7, RGS1, CCL4, IFNG, IL12RB2, IL13RA1, KMO, FPR2, SLC27A2,* and *C*3;
b) at least two reporter probes capable of binding to complementary DNA (cDNA) derived from the transcripts, which are suitable to be detected in a quantitative reverse transcription Polymerase Chain Reaction (qRT-PCR), and/or
   at least two labelled second antibodies, each of which specifically binding to one of the first antibodies or molecules, which are designed to be detected in a high-affinity binding assay (immune-/antibody-based assay such as Enzyme Linked Immunosorbent Assay (ELISA)); and optionally,
c) at least two reference samples.

For example, the kit may comprise naturally occurring or chemically synthesized molecules capable of specifically binding or hybridizing to the marker proteins or the transcripts (mRNA) of the marker genes. Such molecules may be selected from the group comprising a peptide, an antibody or a fragment thereof, an aptamer (peptide or oligonucleotide), and an oligonucleotide (primer). Some molecules may be labelled with a detectable label, such as a luminescent, fluorescent or radioactive group.

For example, said kit may comprise at least one set of oligonucleotide primers selected from the group consisting of
a) a set of oligonucleotide primers comprising the nucleic acid sequences according to SEQ ID NO:1 and SEQ ID NO:2;
b) a set of oligonucleotide primers comprising the nucleic acid sequences according to SEQ ID NO:3 and SEQ ID NO:4
c) a set of oligonucleotide primers comprising the nucleic acid sequences according to SEQ ID NO:5 and SEQ ID NO:6;
d) a set of oligonucleotide primers comprising the nucleic acid sequences according to SEQ ID NO:7 and SEQ ID NO:8;
e) a set of oligonucleotide primers comprising the nucleic acid sequences according to SEQ ID NO:9 and SEQ ID NO:10;
f) a set of oligonucleotide primers comprising the nucleic acid sequences according to SEQ ID NO:11 and SEQ ID NO:12;
g) a set of oligonucleotide primers comprising the nucleic acid sequences according to SEQ ID NO:13 and SEQ ID NO:14
h) a set of oligonucleotide primers comprising the nucleic acid sequences according to SEQ ID NO:15 and SEQ ID NO:16;
i) a set of oligonucleotide primers comprising the nucleic acid sequences according to SEQ ID NO:17 and SEQ ID NO:18;
j) a set of oligonucleotide primers comprising the nucleic acid sequences according to SEQ ID NO:19 and SEQ ID NO:20;
k) a set of oligonucleotide primers comprising the nucleic acid sequences according to SEQ ID NO:21 and SEQ ID NO:22;
l) a set of oligonucleotide primers comprising the nucleic acid sequences according to SEQ ID NO:23 and SEQ ID NO:24;
m) a set of oligonucleotide primers comprising the nucleic acid sequences according to SEQ ID NO:25 and SEQ ID NO:26;
n) a set of oligonucleotide primers comprising the nucleic acid sequences according to SEQ ID NO:27 and SEQ ID NO:28;
o) a set of oligonucleotide primers comprising the nucleic acid sequences according to SEQ ID NO:29 and SEQ ID NO:30;
p) nucleic acid molecules, the polynucleotide sequence of which is at least 90%, preferably 95%, identical to the nucleotide sequence of a oligonucleotide primer of any of a) to o), and which is capable of binding to complementary DNA (cDNA) derived from the transcripts of a gene coding for at least one marker protein selected from the group consisting of the proteins listed in Table 1;
q) nucleic acid molecules, the complementary strand of which hybridizes to a nucleic acid molecule of any of a) to p) under stringent conditions;
r) nucleic acid molecules, the nucleotide sequence of which is complementary to the nucleotide sequence of a nucleic acid molecule of any of a) to q).

The primer sequences are shown in Table 2 (SEQ ID NOS refer to the primers for amplifying the human marker genes).

The invention further concerns a method for determining the response to at least one pharmaceutical compound able to correct a dysfunctional DISC1 protein pathway or disturbed dopamine homeostasis, wherein the expression levels of at least two marker genes are determined and compared according to steps a) and b) of the method according to claim 1, and wherein the measured expression levels indicate that the response to the pharmaceutical compound is positive if each aberrant expression level of said marker genes is normalized or at least improved. Accordingly, a method for monitoring the therapeutic efficacy of a pharmaceutical compound in an individual having a mental disorder is provided, comprising a comparison of a current expression level of the marker genes present in a body tissue or fluid sample of said individual after administration of the pharmaceutical compound with at least one sample taken earlier from the same individual, e.g., prior to commencement of the therapy, and/or from the same individual at an earlier stage of therapy. If the expression levels of the marker genes are changed by the application of the pharmaceutical compound in a way that it is at least partially normalized towards the respective average expression level of a healthy population (i.e. reference (standard) expression level), the therapeutic response to the pharmaceutical compound is positive. That is, a therapy is effective if the difference between the current expression levels and the threshold values is decreased in relation to the difference between the earlier expression levels and the threshold values. With this method according to the invention it is possible to analyse the efficacy of existing pharmaceutical compounds or those that are not developed using a transgenic animal as described below.

For example, the protein pathology of a subset of sporadic mental disorders as outlined above can be modeled by means of a transgenic animal (e.g. by modestly overexpressing the non-mutant full length human DISC1 transgene: tgDISC1 rat) and presents a novel pharmacological target in mental illness drug discovery. The concept of "reverse translation" assumes the existence of subsets of mental disorders from the outset and thus starts marker search with a biologically defined subset rather than a mix of heterogeneous cases merely defined by clinical diagnosis. Genetic or protein pathology of patients/families can be modeled in animals and then markers can be identified in this animal model. Markers can be "reverse translated" to sporadic patients in order to define those biological subsets ante mortem, i.e. in live patients. Animal model, marker and patient subsets can then be paired in order to develop tailored therapies.

For example, a transgenic rat model modestly overexpressing the full-length DISC1 transgene (named "tgDISC1 rat"), shows phenotypes consistent with a significant role of DISC1 misassembly in a subset of sporadic mental disorders. The tgDISC1 rat displays mainly perinuclear DISC1 aggregates in neurons. Furthermore, the tgDISC1 rat shows a robust signature of behavioural phenotypes that includes amphetamine supersensitivity, hyperexploratory behaviour, rotarod deficits, as well as aberrant dopamine neuroanatomy and neurochemistry, all pointing to changes in dopamine (DA) neurotransmission. Elevated cytosolic dopamine causes an increase in DISC1 multimerization, insolubility and complexing with the dopamine transporter, suggesting a physiological mechanism linking DISC1 assembly and dopamine homeostasis. DISC1 protein pathology and its interaction with dopamine homeostasis is a novel cellular mechanism that is relevant for behavioural control and may have a role in mental disorders (Trossbach 2016). Neuroanatomical analysis revealed a reduced density of dopaminergic neurons in the substantia nigra and reduced dopaminergic fibres in the striatum of the transgenic rat. Parvalbumin-positive interneuron occurrence in the somatosensory cortex was shifted from layers II/III to V/VI, and the number of calbindin-positive interneurons was slightly decreased. Reduced corpus callosum thickness confirmed trend-level observations from *in vivo* MRI and voxel-wise tensor based morphometry. These neuroanatomical changes help explain functional phenotypes of this animal model, some of which resemble changes observed in human schizophrenia post mortem brain tissues. DISC1 overexpression or misassembly can account for a variety of seemingly unrelated morphological phenotypes and thus provides a possible explanation for findings observed in sporadic schizophrenia patients (Hamburg et al. 2016).

Accordingly, the tgDISC1 rat reflects neuropathological features of a subset of sporadic cases with CMI and has behavioral (amphetamine sensitization) and biochemical (D2R high switch) features very similar to human patients with schizophrenia, and therefore exhibits high face validity. On a molecular mechanistic level, DISC1 missassembly seems to modulate dopamine homeostasis so that the tgDISC1 rat is a useful model for a subset of sporadic CMI, advancing biological diagnostics and therapy. Further, DISC1opathies define a biology-based category of human mental disorders with involvement of the dopamine system that can be modeled in animals, i.e. in the tgDISC1 rat. The tgDISC1 rat represents a subset of patients with schizophrenia (or other mental illnesses), termed "DISC1opathies".

It is conceivable that human induced pluripotent stem (iPS) cells modestly overexpressing a similar DISC1 transgene, and differentiated to various cells or brain organoids, including human PBMC subpopulations, might also be used for the purpose of identifying possible markers and patient-tailored therapies but with the shortcoming that these cell systems are not amenable to behavioural testing paradigms. However, for specific applications, at least one human induced pluripotent stem (iPS) cell may be provided which is able to stably express a modified gene coding for human DISC1 protein, wherein the expression level of the modified gene is higher than that of the respective wild-type gene and thus results in the formation of aggregates of the DISC1 protein within the cell. For example, such iPS cell can also be used for in the identification and analysis of marker proteins or genes for diagnosing mental disorders in human individuals.

With the concept of reverse translation, a distinct marker set can be identified, that may not be complete but that is sufficient to allow fairly specific blood diagnostics of DISC1 opathies. The blood test may be used to identify patients that may profit from (future) curative pharmacotherapies also effective in the tgDISC1 rat. However, they may also profit from existing merely symptomatic pharmacotherapies targeting neurotransmitter systems by indicating which patient subsets are likely to respond to a dopamine homeostasis-modifying drug. For example, a patient that has been tested positive for a DISC1 opathy may receive one particular dopamine-homeostasis reinstating drug with priority rather than testing various drugs based on clinical guesses as is current clinical practice.

Curative drugs are defined as drugs that target the biological cause, for example DISC1 protein pathology, of a mental disorder whereas symptomatic drugs are drugs that target one downstream consequence of the biological causes, for example aberrant dopamine homeostasis. Curative drugs are advantageous because they eventually eliminate all downstream aberrances, rather than only one.

It is possible that an aberrant DISC1 pathway is also encountered in human individuals that are not considered clinically sick. This may be due to a variety of causes also termed resilience factors. These individuals, however, may reveal subtle cognitive impairments upon closer inspection that may improve with suitable drugs which would then be called cognitive enhancement rather than pharmacotherapy. That is, a potential therapy for clinically sick patients might also be used as cognitive enhancer for healthy individuals.

The term "marker gene" as used herein refers to a distinctive gene coding for a distinctive protein or peptide (herein referred to as "marker protein") suitable to be used as an indicator of a biological, biochemical, or physiological process, event, or condition within a body, tissue, or cell. For example, a marker gene can be used to detect at least one biological, biochemical, or physiological symptom of a disease by detection of its transcript or protein. In this context, "distinctive" means that the marker gene or marker protein is accessible to be detected via a physical, physico-chemical, or electro-physical detection method, either directly or indirectly by means of at least one detectable (labelled) compound or composition. The marker gene can be detected either by detecting the marker protein or by detecting the transcripts (mRNA, or indirectly: cDNA) of the marker gene. If specific marker proteins are named herein, all derivatives thereof comprising processed proteins or splice variants, i.e. molecules derived from the original gene or protein indicated, shall be included.

The term "marker" as used herein includes both the marker gene and the marker protein.

The term "expression level" (or "expression level of the marker gene") as used herein refers to the amount of transcript (mRNA) of a gene coding for a specific peptide or protein (transcript level), or the amount of specific peptide or protein derived from this gene (protein level), within a body, tissue, cell, or fluid sample. The expression level can be measured, for example, by quantitative determination of the amount of either mRNA (or cDNA) or translated protein within a sample.

The term "aberrant expression level" as used herein refers to an abnormal expression level of a marker gene, which significantly differs from the average expression level of the same marker gene in a healthy population and is involved in the clinical manifestation of a disease and/or represents a symptom of a disease, e.g., a mental disorder.

The invention is further exemplarily described in detail with reference to the figures.

### Brief description of the figures

**Figure 1** shows two alternative, complementary and selective (incomplete) depictions of the DISC1 pathway, demonstrating that the DISC1 protein interacts with many proteins and signaling pathways that have independently been described for mental illness or other chronic brain disease conditions.

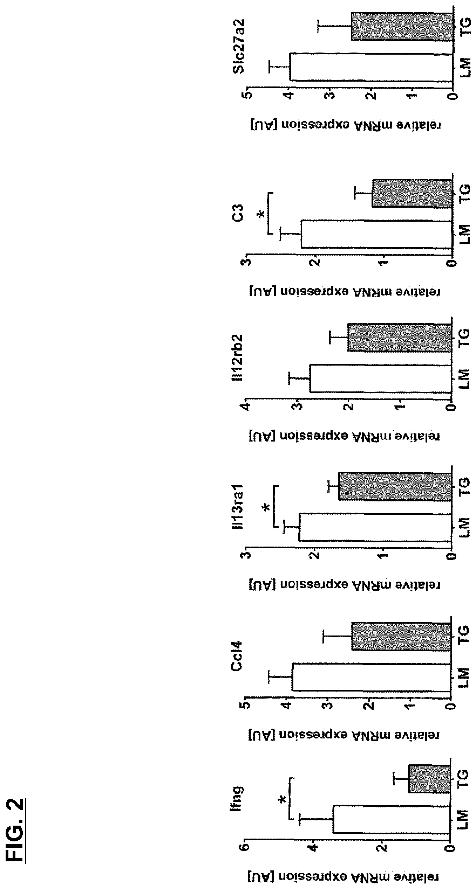

**Figure 2** shows bar diagrams representing an independent validation of a selection of markers from Table 1 in the tgDISC1 rat (TG, gray) vs. non-transgenic littermates (LM, white) using quantitative polymerase chain reaction (qPCR). Marker names abbreviated, for full name, see Table 1.

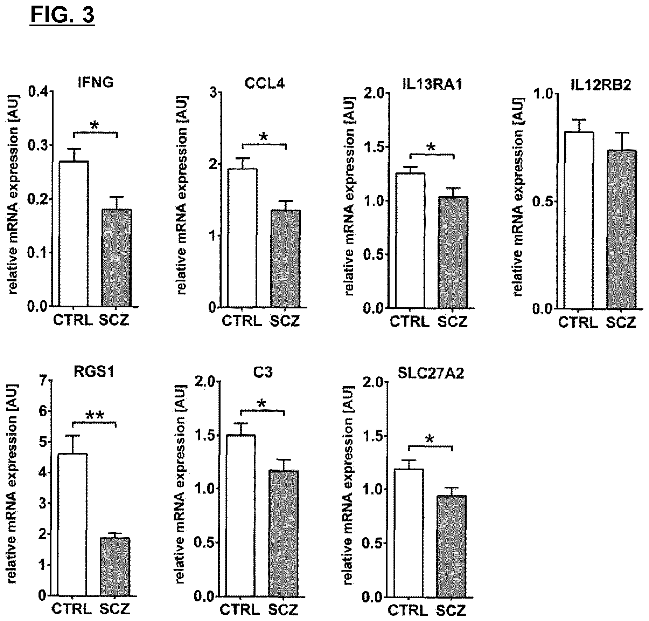

**Figure 3** shows bar diagrams representing a screening of a selection of markers from Table 1 in a population of schizophrenia patients vs. controls (n = 20 for each group); cohort of patients with schizophrenia (SCZ, gray) vs. healthy controls (CTRL, white). Marker names abbreviated, for full name, see Table 1.

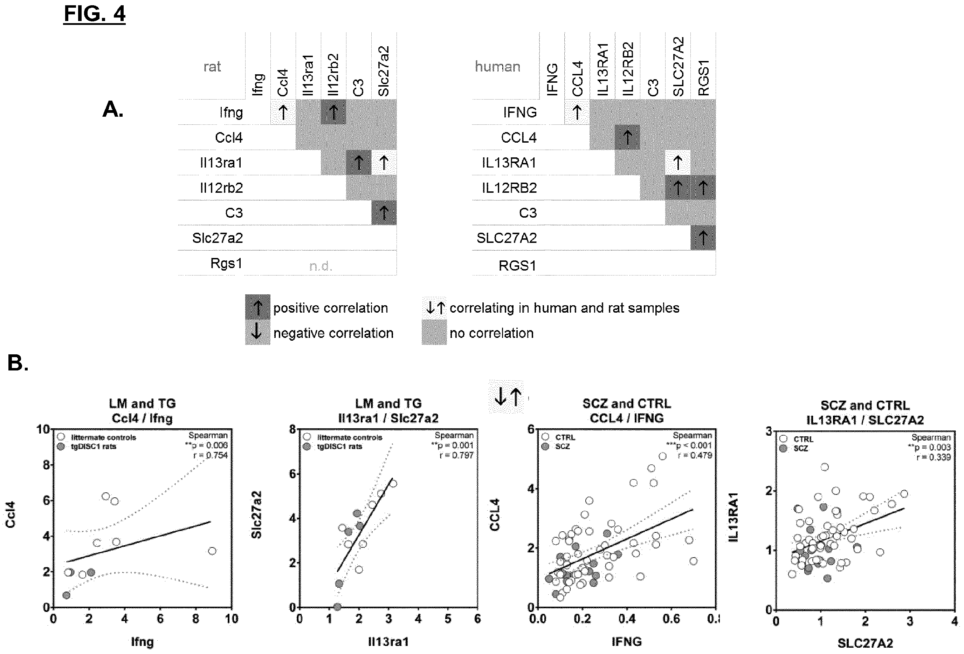

**Figure 4** shows graphical representations demonstrating the correlation of different markers in the tgDISC1 rat and schizophrenia patients. A. Correlation matrix of markers in the rat (left) and human (right). B. Selective depiction of single correlations appearing similar in the transgenic tgDISC1 rat (TG) vs. non-transgenic littermates (LM), and schizophrenia patients (SCZ) vs. healthy controls (CTRL).

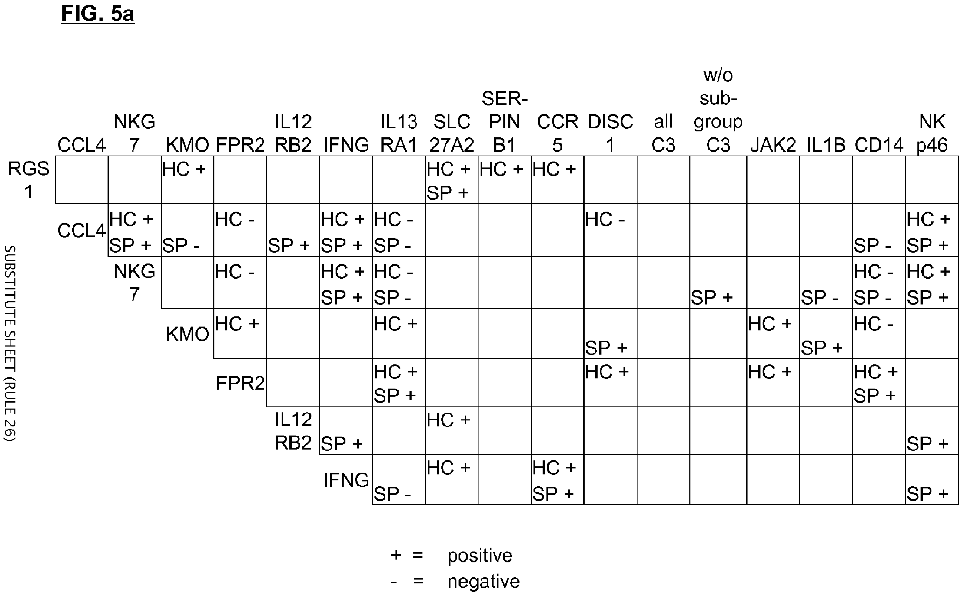

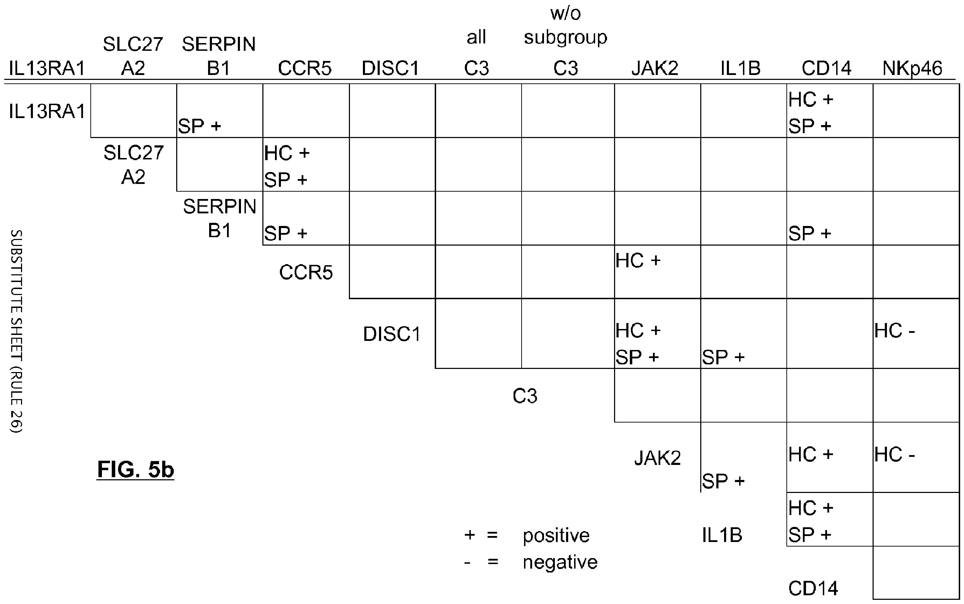

**Figure 5** shows a table of a Spearman correlation (non-parametric) of human markers (see Table 1) demonstrating that single correlations either exist between patients and controls, or only for patients or controls demonstrating the disruption or pathological creation and stabilization of regulated networks of markers (all data analyzed without outliers).

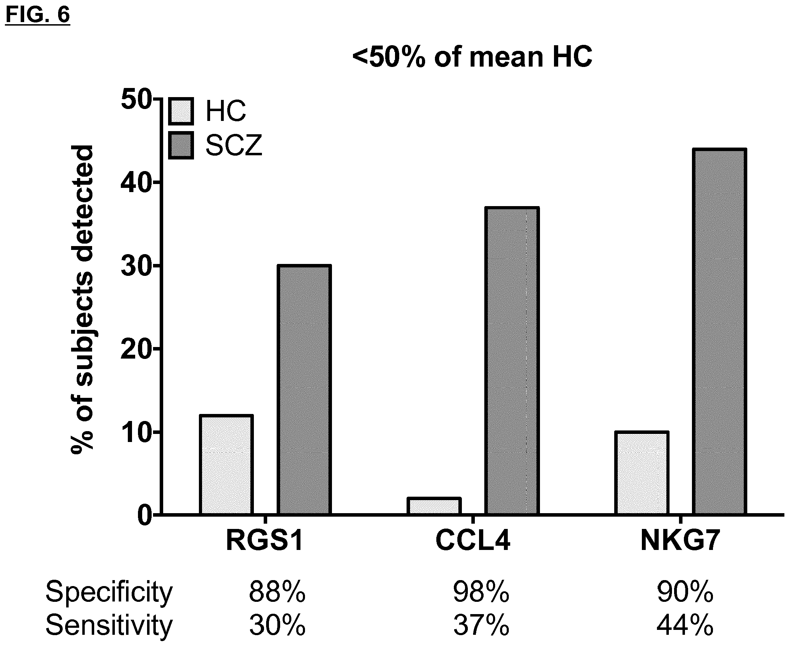

**Figure 6** shows a bar diagram demonstrating detection specificity and sensitivity related to the clinical diagnosis of schizophrenia (SCZ, dark grey; healthy controls (HC), light grey) when a selection of single markers from Table 1 is investigated. The threshold was defined at being below 50% of the average of the healthy control group.

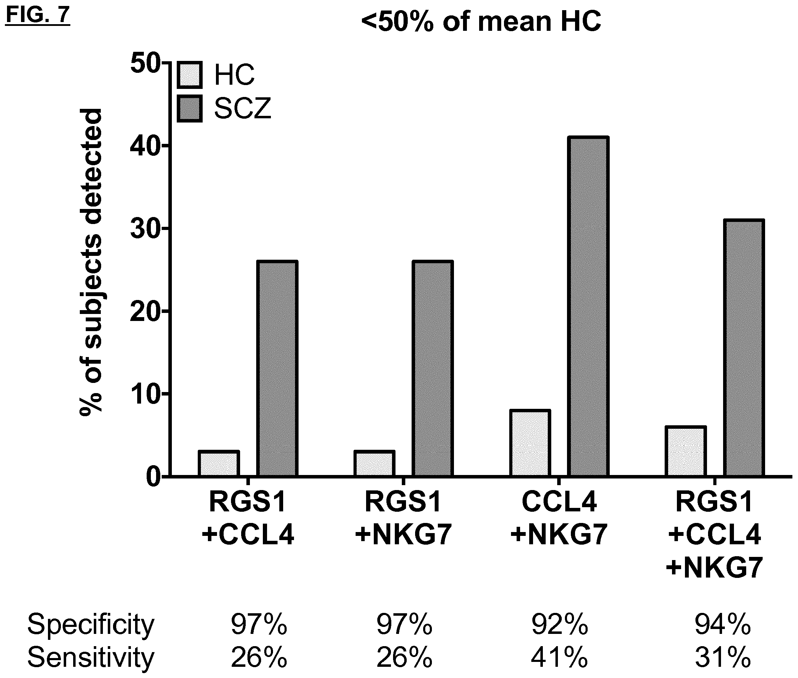

**Figure 7** shows a bar diagram demonstrating detection specificity and sensitivity related to the clinical diagnosis of schizophrenia (SCZ, dark grey; healthy controls (HC), light grey) when a selection of a combination of two or three markers from Table 1 is investigated. The threshold was defined at being below 50% of the average of the healthy control group.

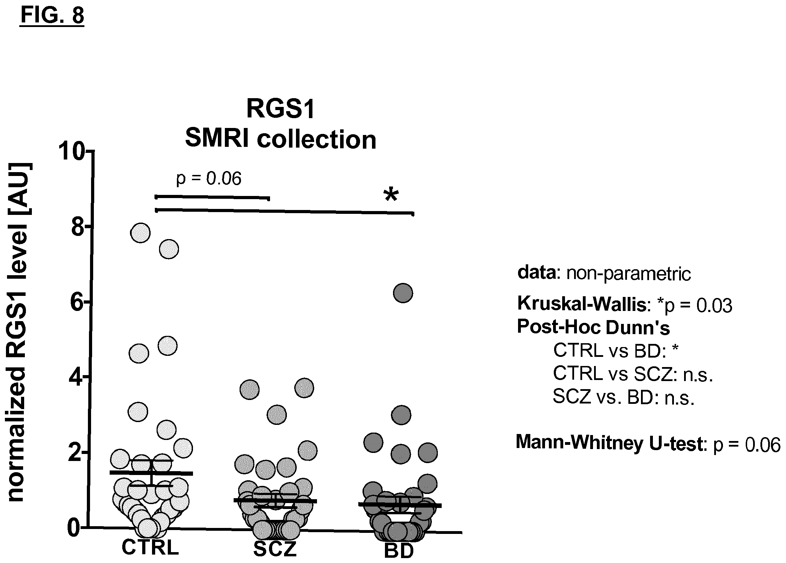

**Figure 8** shows a graph representing normalized expression levels (brain) of *RGS1* transcripts in controls (CTRL), schizophrenia (SCZ) and Bipolar Disorder (BP) samples.

Description of exemplary and preferred embodiments of the invention

### Subjects and classifications

Control subjects and patients diagnosed with schizophrenia were part of a clinical study as described by Warbrick et al. (2011) and Trossbach et al. (2014).

### Animals

Animal experiments were executed in conformity with the German Animal Protection Law and were authorized by local authorities (LANUV NRW, Recklinghausen, Germany). Experiments were performed with transgenic Sprague Dawley rats overexpressing full-length, non-mutant DISC1 carrying the polymorphisms L607F (rs6675281) and S704C (rs821616) (tgDISC1 rat; Trossbach et al., 2016) and non-transgenic littermates. Male tgDISC1 and control rats were bred at the Heinrich Heine University Düsseldorf, Animal Facility, Germany. Animals were housed three animals per cage under standard laboratory conditions with lights on from 0700 hours to 1900 hours and with water and food provided ad libitum. Blood extraction and preparation of lymphocytes was performed with adult tgDISC1 rats and littermate controls at the age of 8-9 months.

### Preparation of lymphocytes from blood

Anaesthetized rats underwent a heart puncture to harvest a minimum of 8 mL of blood. Rat lymphocytes were prepared with the Ficoll-Paque Premium 1.084 solution (GE Healthcare, Little Chalfont, United Kingdom) according to manufacturer's instructions. Preparation of human lymphocytes was performed with Ficoll-Paque Plus (GE Healthcare, Little Chalfont, UK) as described by Trossbach et al. (2014). Lymphocyte samples were snap-frozen in liquid nitrogen and stored at -80°C until further processing.

### Preparation of RNA and cDNA

RNA of rat and human lymphocytes was prepared utilizing the RNeasy Mini Kit according to manufacturer's guidelines. Residual genomic DNA was digested on column by the RNase-free DNasel Set (both Qiagen, Hilden, Germany). RNA was diluted to a concentration of 100 ng/µL and 1 µg was used as input for the production of cDNA with the RevertAid First Strand Synthesis Kit in a total of 20 µL utilizing the random hexamer primers provided by the kit (Thermo Fisher Scientific, Waltham, MA, USA). The resulting cDNA was diluted 1:50, 1:25 or 1:50 dependent on PCR results as indicated in Table 1 and 5 µL were used as template input.

### Gene expression profiling

Total RNA preparations were checked for RNA integrity by Agilent 2100 Bioanalyzer quality control. All samples in this study showed high quality RNA Integrity Numbers (RIN >9). RNA was further analysed by photometric Nanodrop measurement and quantified by fluorometric Qubit RNA assays (Life Technologies). Synthesis of biotin labeled cDNA was performed on ten replicates of each experimental group (DISC1 transgenic (TG) rats and littermate (LM) controls, respectively) according to the manufacturers' protocol (WT Plus Reagent Kit; Affymetrix, Inc). Briefly, 100 ng of total RNA were converted to cDNA. After amplification by in vitro transcription and 2nd cycle synthesis, cDNA was fragmented and biotin labeled by terminal transferase. Finally, end labeled cDNA was hybridized to Affymetrix Rat Gene 2.0 ST Gene Expression Microarrays for 16h at 45 °C, stained by strepatavidin/phycoerythrin conjugate and scanned as described in the manufacturers' protocol.

Three samples (2x TG, 1x LM) did not pass hybridization quality control, two additional samples (3x TG, 2x LM) had to be excluded from further analyses because of abnormal ventricle volume.

Data analyses on 12 Affymetrix CEL files were conducted with GeneSpring GX software (Vers. 12.5; Agilent Technologies). Probes within each probeset were summarized by GeneSprings' ExonRMA16 algorithm after quantile normalization of probe level signal intensities across all samples to reduce inter-array variability (Bolstad et al., 2003). Input data pre-processing was concluded by baseline transformation to the median of all samples. To further improve signal-to-noise ratio, a given probeset had to be expressed above background (i.e. fluorescence signal of a probeset was detected within the 20th and 100th percentiles of the raw signal distribution of a given array) in all replicates in at least one of two, or both conditions to be subsequently analysed in pairwise comparison. Differential gene expression was statistically determined by moderated T-test. The significance threshold was set to p = 0.01.

### Quantitative expression analysis

For the verification of differential expression target primers were tested by PCR using the HotStarTaq (Qiagen, Hilden, Germany). Primer sequences, dilutions and PCR supplements are depicted in **Table 2**. Effective primers were used for quantitative Real Time PCR (qPCR) with the StepOnePlus Real-Time PCR System (Applied Biosystems, Carlsbad, CA, USA) and the Platinum SYBR Green qPCR SuperMix-UDG (Invitrogen, Carlsbad, CA, USA) in MicroAMP Fast Optical 96-Well Reaction Plates (Applied Biosystems, Carlsbad, CA, USA). Depending on the target, 5% Factor Q solution (Qiagen, Hilden, Germany) was added to the mix. QPCR conditions: 10 min at 95°C, 40 cycles of 15 s at 95 °C and 60°C for 1 min. The resulting data were processed with the corresponding StepOne Software v2.3 (Thermo Fisher Scientific, Waltham, MA, USA). The expression of the respective target was normalized to the expression level of the housekeeping gene Actin (rat) or ARF1 (human), as well as against a rat or human control cDNA per plate to minimize variances between runs.

As shown in **Figure 2****,** the expression level (relative mRNA expression) of each of the marker genes *Ifng, CcI4, II13ra1, II12rb2, C3, and Slc27a2* from Table 1 is significantly decreased in the tgDISC1 rat (TG, gray) in relation to non-transgenic littermates (LM, white). This result indicates that the decreased expression level of these marker genes observed in the microarray analysis (Table 1) can be repeated by an independent detection method (quantitative polymerase chain reaction; qPCR). Marker genes *Ifng, CcI4, Il13ra1, Il12rb2, C3, and Slc27a2* are all downregulated, i.e. the transcript level is decreased relative to the reference (standard) level of the "healthy" littermates. That is, the lower expression level of each tested marker gene in the transgenic rat relative to the expression level of the respective marker gene in the non-transgenic control is indicative of dysfunctional DISC1 protein pathway or disturbed dopamine homeostasis. The altered expression level can thus be deemed to be indicative to neuropathological and biochemical features very similar to human patients with schizophrenia.

**Figure 3** shows a screening of marker genes *IFNG, CCL4, IL13RA1, IL12RB2, RGS1, C3, and SLC27A2* from Table 1 in a population of schizophrenia patients (SCZ, gray) vs. healthy controls (CTRL, white), demonstrating that the decreased expression levels (relative mRNA expression) of the same markers in the tgDISC1 model are also observed in a cohort of patients with schizophrenia. Marker genes *IFNG, CCL4, IL13RA1, IL12RB2, RGS1, C3, and SLC27A2* are all downregulated, i.e. the transcript level is decreased relative to the reference (standard) level of the healthy controls. Accordingly, the altered expression level can be deemed to be indicative to schizophrenia in human individuals.

**Figure 4** shows correlations of different markers in tgDISC1 rats and human individuals, demonstrating similarity between the rat system and the human system. The alterations of the expression levels of the tested markers relative to the respective reference expression levels are similar in the tgDISC1 rat and schizophrenia patients. Thus, transfer of the principles and mechanisms observed in the rat system to the human system is reasonable.

**Figure 5** shows a correlation table of human markers demonstrating that single correlations either exist between patients and controls, or only for patients or controls. The cross-correlations between single markers show that disease can either disrupt existing correlating networks or stabilize new (pathological) ones and demonstrates that the identified markers according to Table 1 are functionally interconnected.

**Figures 6** **and** **7** demonstrate the detection specificity and sensitivity related to the clinical diagnosis of schizophrenia when a selection of single markers from Table 1 (RGS1, CCL4, and NKG7; Figure 6) or a selection of a combination of two or three markers from Table 1 (RGS1 + CCL4 and/or NKG7, and CCL4 + NKG7; Figure 7) is investigated. Basically it is demonstrated that specificity of the method according to the invention is very high while sensitivity is rather low. However, in the method according to the invention specificity is more important than sensitivity since the method is provided for a sub-group of patients only and thus low sensitivity relating to the all-comprising clinical diagnosis is expected and can therefore be neglected. **Figure 7** shows that the combination of two or more markers can significantly increase specificity of the method according to the invention, cf. marker gene RGS1 (alone: 88% => + CCL4 and/or NKG7: 94% / 97%).

**Figure 8** shows that, surprisingly, *RGS1* expression in brain is clearly decreased in patients (Schizophrenia and Bipolar Disorder) compared to healthy controls. *RGS1* is correlated to cognitive endophenotypes of patients and seems to be the best marker for diagnosing related diseases. *RGS1* is significantly correlated to attention and memory in the Digital Symbol Test (DSST) indicating high clinical relevance of this marker to measurable cognitive deficiencies (data not shown). The decrease in *RGS1* expression is not due to a decreased number of macrophages where it is expressed and which would correspond to the cell lineage where microglia is also derived from, the only cell type of the brain expressing RGS1 (data not shown). *RGS1,* preferably in conjunction with an NK cell marker, therefore seems to be advantageous to diagnose the patient subsets with credibility, plausibility and specificity.

Accordingly, RGS1 seems to crystallize as the most important marker compared to the other markers listed in Table 1. However, *RGS1* expression levels seem also changed in diseases like melanoma, multiple sclerosis or others, so that at least a second marker can be beneficial. For example, the NK cell markers NKG7 or CCL4 may be of particular importance (but interchangeable). In the rat NKG7 and CCL4 indicate a decrease in expression levels, in humans it is not clear whether they are decreased due to a decrease in NK cell number, a decrease in expression per NK cell, or both. In summary, diagnostics could be prioritized to RGS1 and one or two NK cell markers such as NKG7 and CCL4, cf. **Figure 7**. In patients, NK cell markers seem decreased due to a decrease in NK cell numbers, but a simultaneous decrease in expression levels cannot be excluded (data not shown).

### Non-patent literature:

1. Bolstad et al. (2003): A comparison of normalization methods for high density oligonucleotide array data based on variance and bias. Bioinformatics, 19, 2, 185-193, 2003.
2. Chan et al. (2015): "Development of a blood-based molecular biomarker test for identification of schizophrenia before disease onset." Translational Psychiatry (2015) 5, e601; doi:10.1038/tp.2015.91; published online 14 July 2015.
3. Dean (2011): "Dissecting the Syndrome of Schizophrenia: Progress toward Clinically Useful Biomarkers." Schizophrenia Research and Treatment Volume 2011, Article ID 614730, 10 pages, doi:10.1155/2011/614730.
4. Hamburg et al. (2016): "Simultaneous effects on parvalbumin-interneuron and dopaminergic system development in a transgenic rat model for sporadic schizophrenia." Scientific Reports | 6:34946 | DOI: 10.1038/srep34946.
5. Hennah & Porteous (2009): "The DISC1 pathway modulates expression of neurodevelopmental, synaptogenic and sensory perception genes." PLoS ONE 2009; 4(3):e4906
6. Howes & Kapur (2009): "The dopamine hypothesis of schizophrenia: version III--the final common pathway." Schizophr Bull. 2009 May; 35(3):549-62. doi: 10.1093/schbul/sbp006. Epub 2009 Mar 26.
7. Korth (2009): "DISCopathies: brain disorders related to DISC1 dysfunction." Rev Neurosci 2009; 20(5-6):321-30
8. Korth (2012): "Aggregated proteins in schizophrenia and other chronic mental diseases: DISC1opathies." Prion 6(2):134-41.
9. Korth (2012): "Aggregated proteins in schizophrenia and other chronic mental diseases." Prion 6:2, 1-8. April/May/June 2012.
10. Soares et al. (2011): "DISC1: Structure, Function, and Therapeutic Potential for Major Mental Illness." ACS Chem Neurosci. 2011 Nov 16;2(11):609-632. Epub 2011 Aug 5.
11. Trossbach et al. (2014): "Peripheral DISC1 protein levels as a trait marker for schizophrenia and modulating effects of nicotine." Behav Brain Res 275C: 176-182.
12. Trossbach et al. (2016): "Misassembly of full-length Disrupted-in-Schizophrenia 1 protein is linked to altered dopamine homeostasis and behavioral deficits." Molecular Psychiatry (2016), 1-12.
13. Warbrick et al. (2011): "Direction and magnitude of nicotine effects on the fMRI BOLD response are related to nicotine effects on behavioral performance." Psychopharmacology (Berl) 215(2): 333-344.

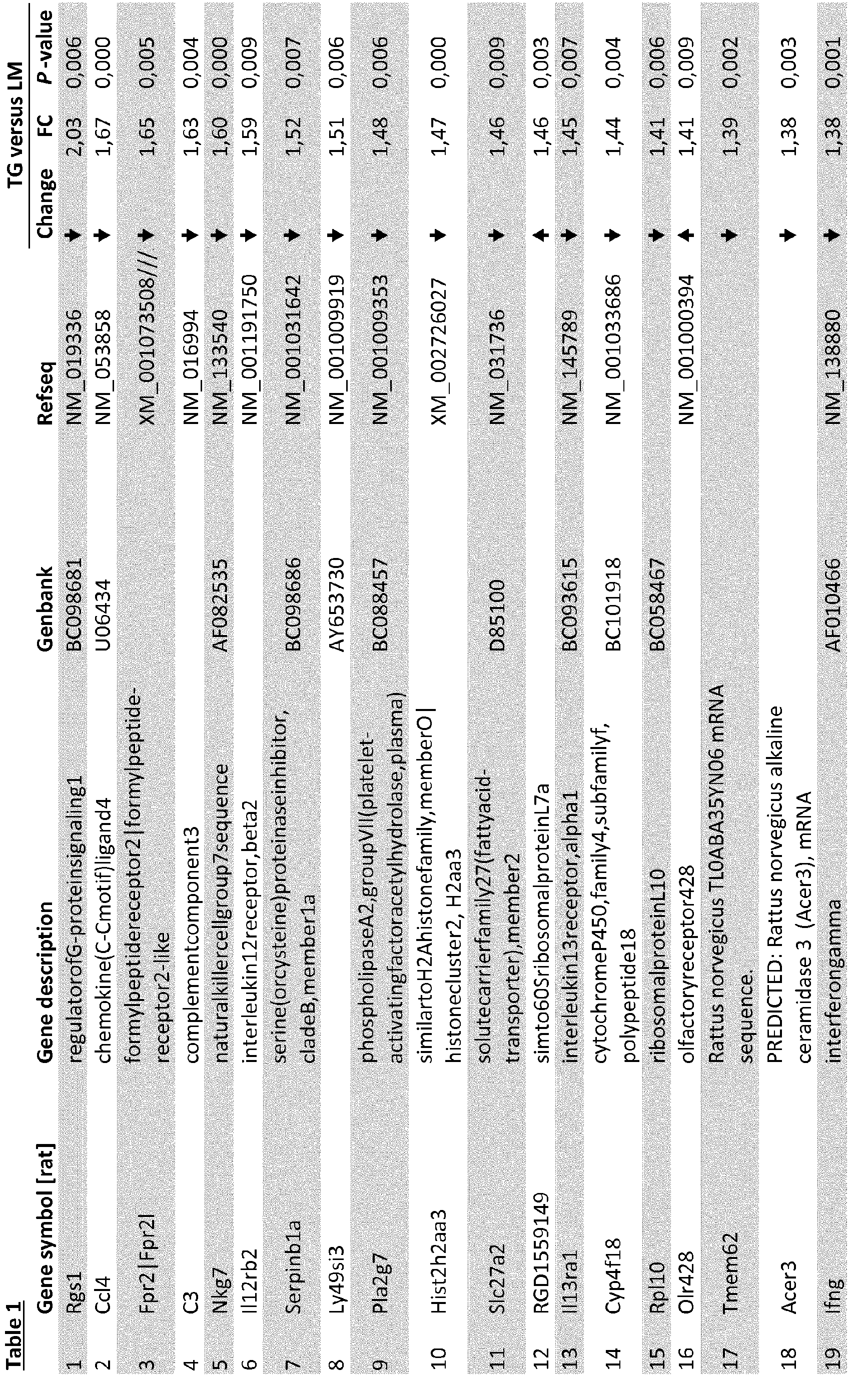

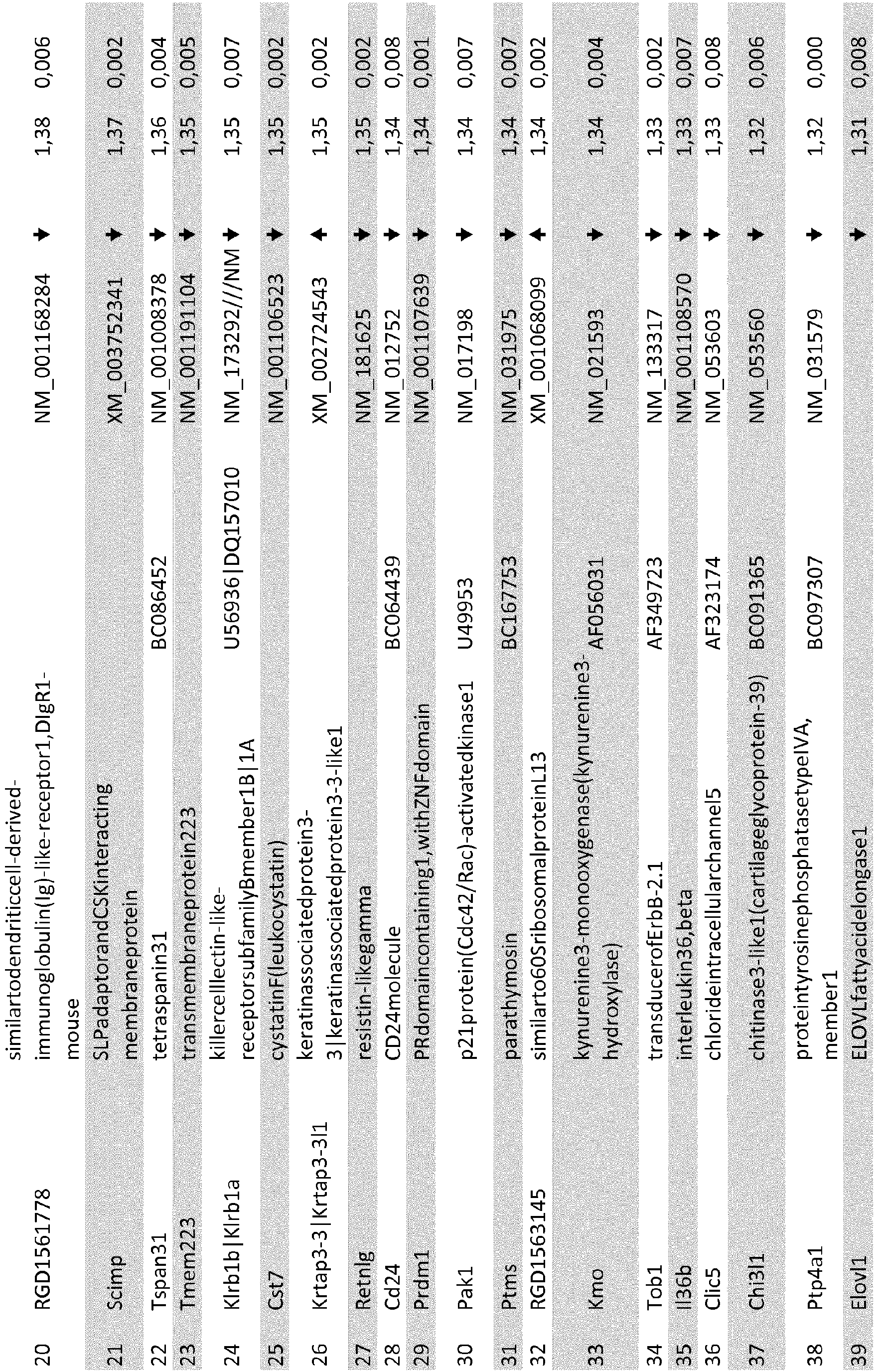

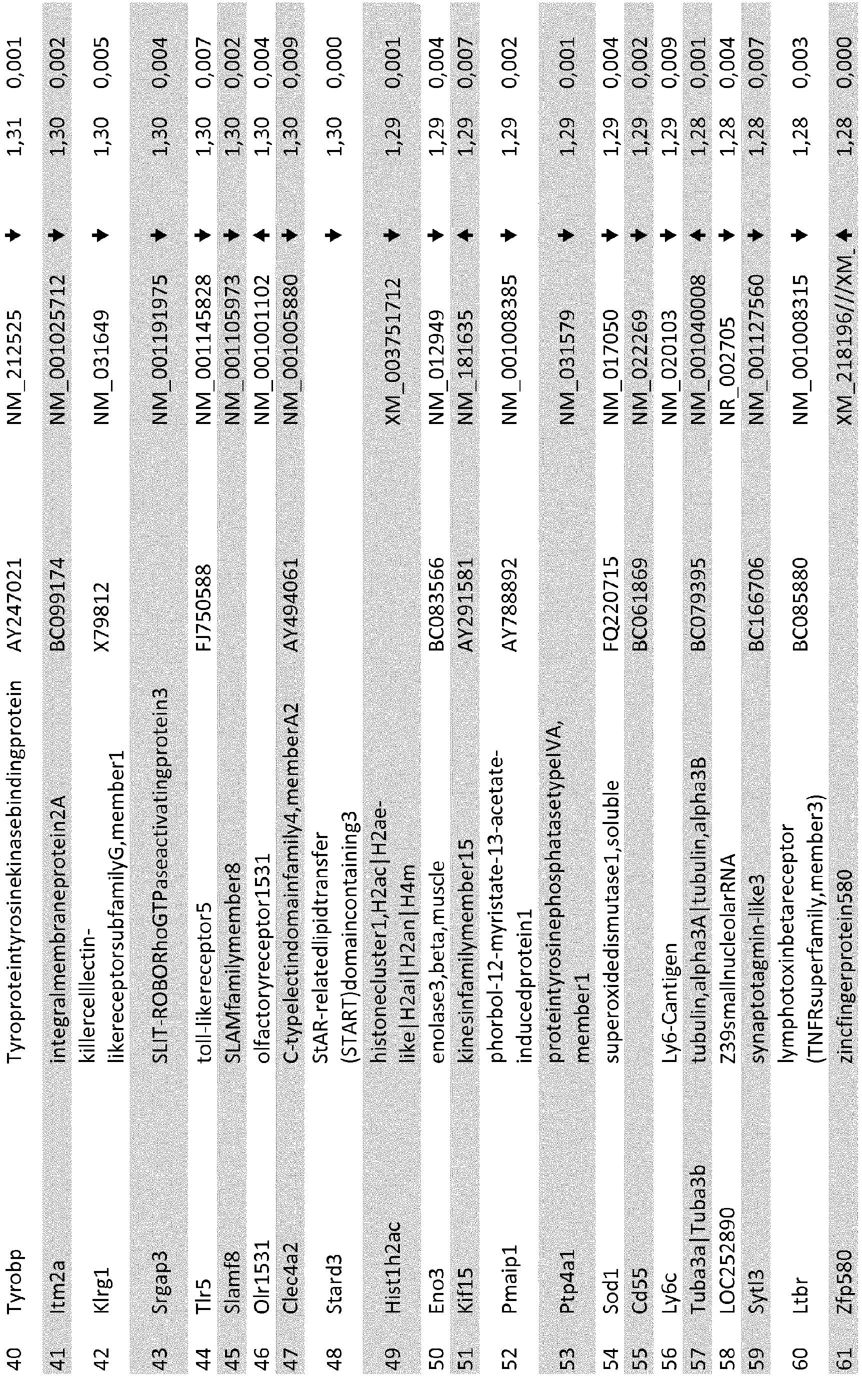

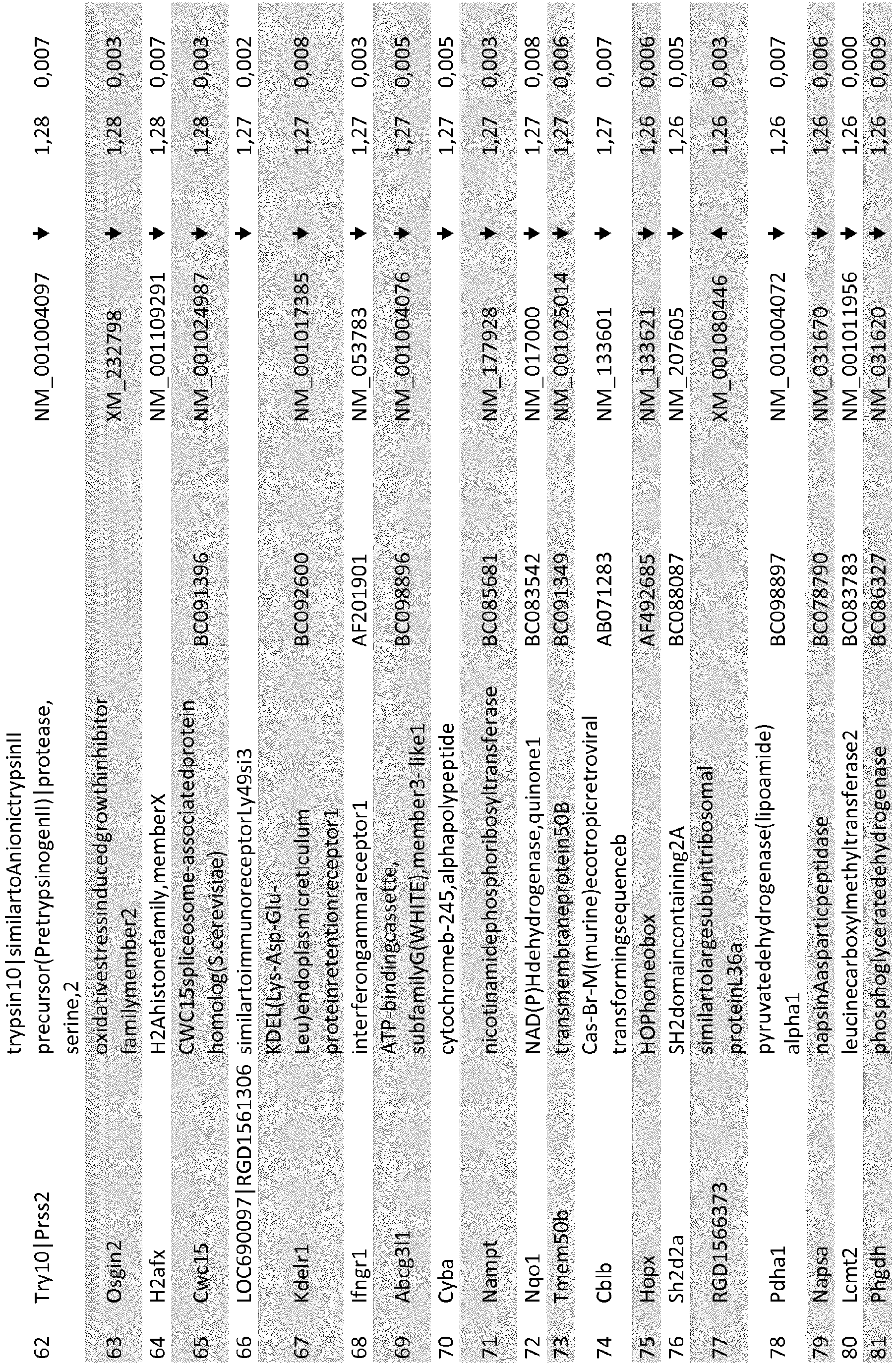

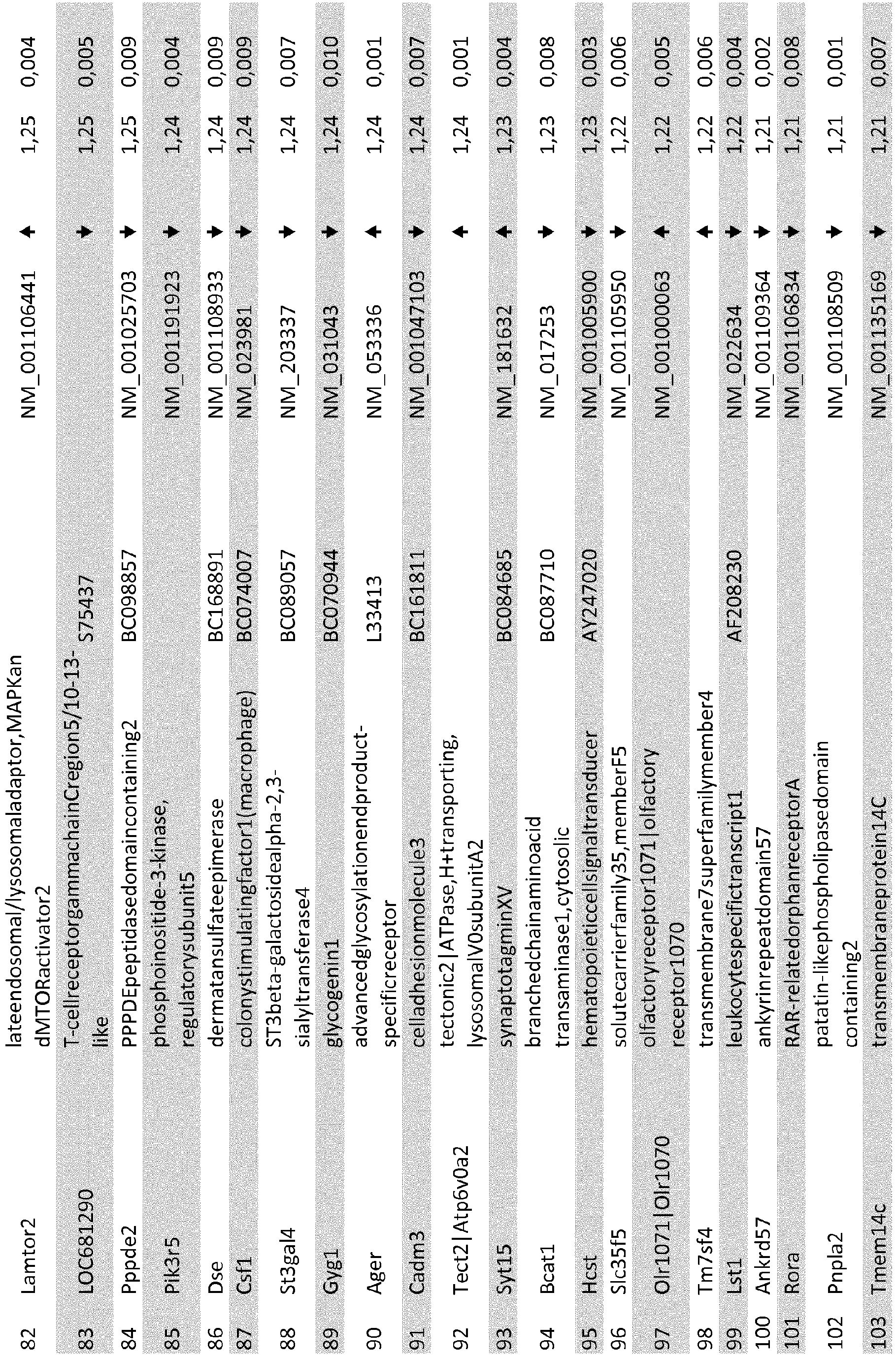

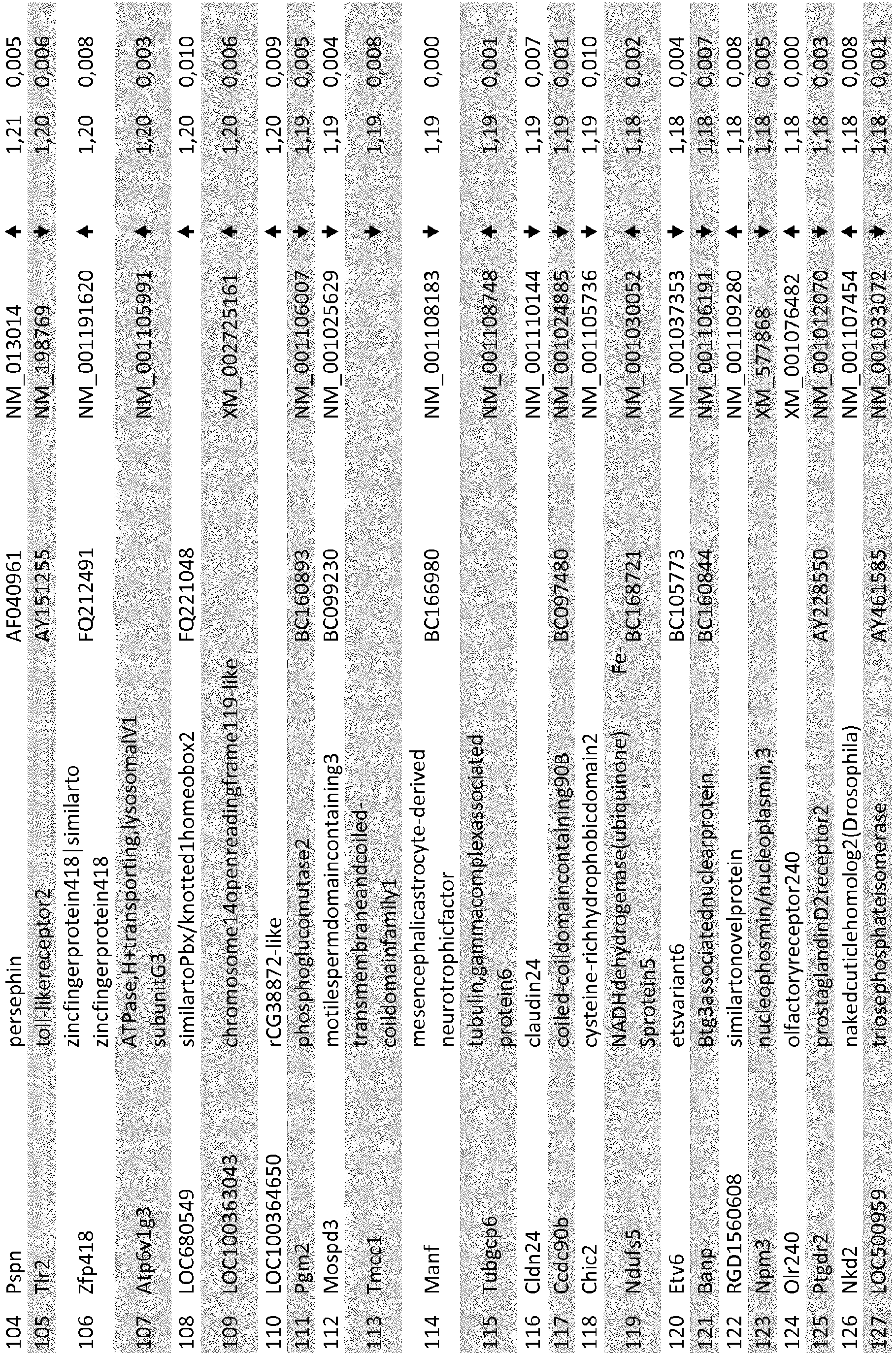

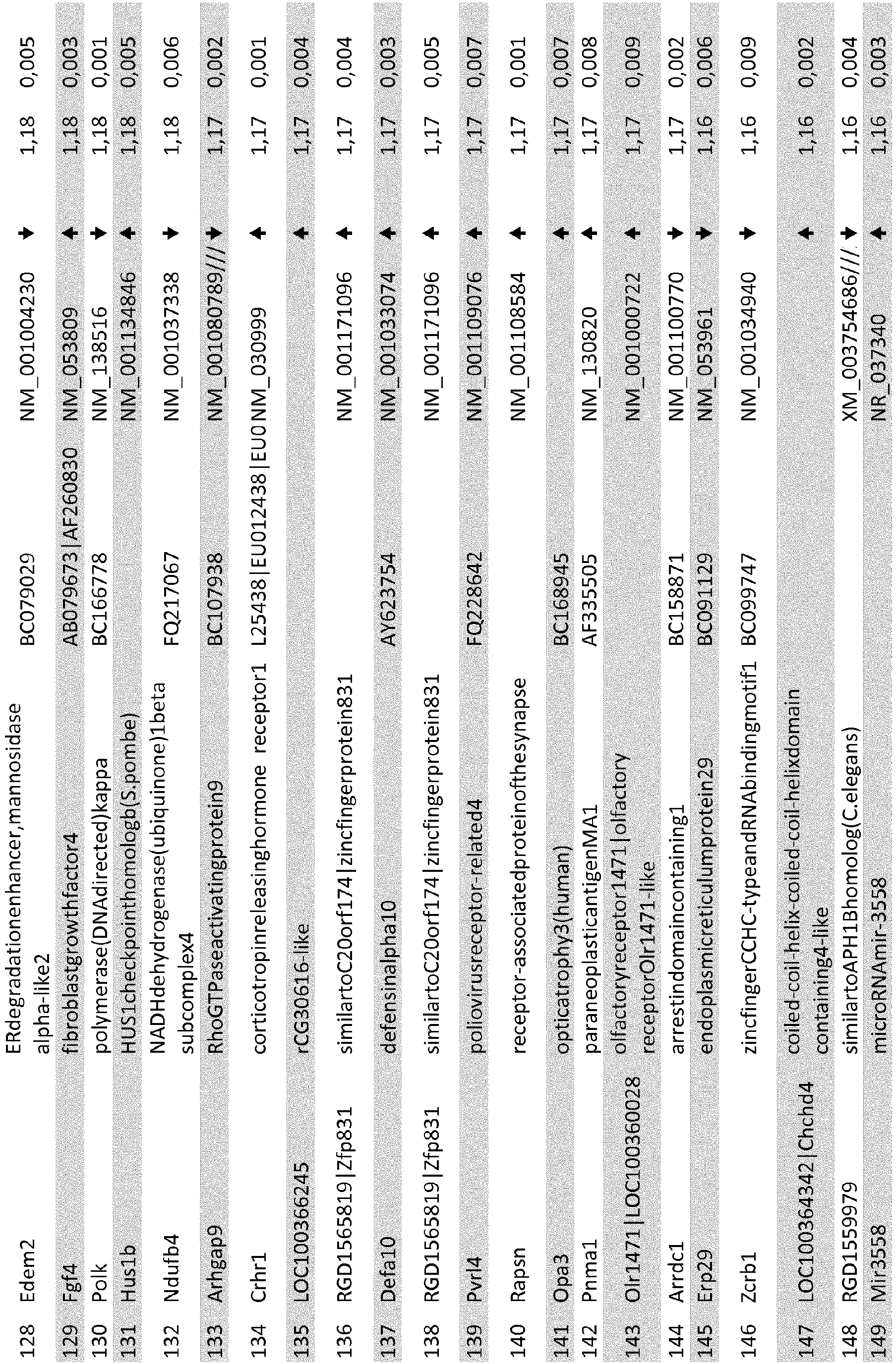

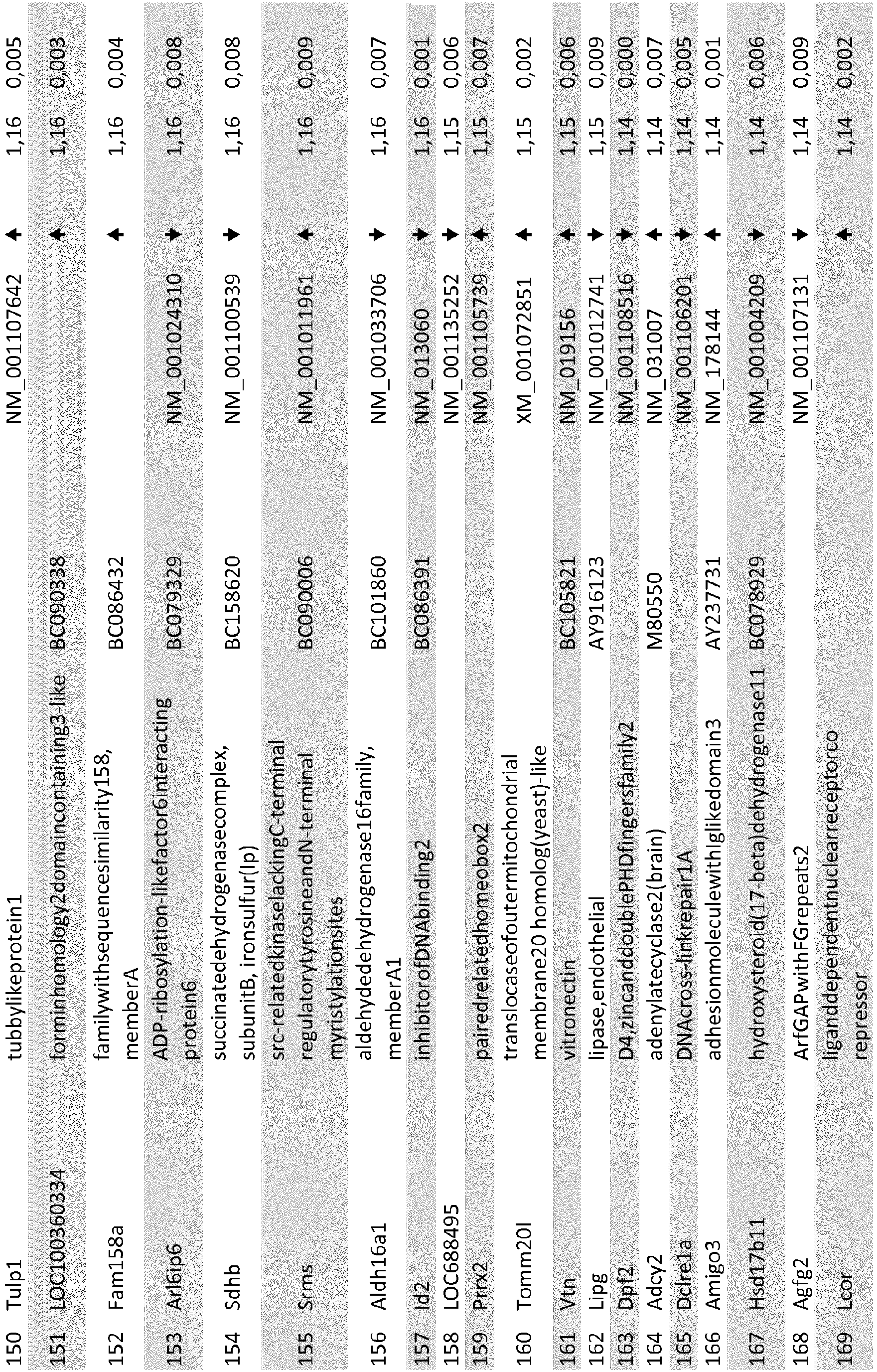

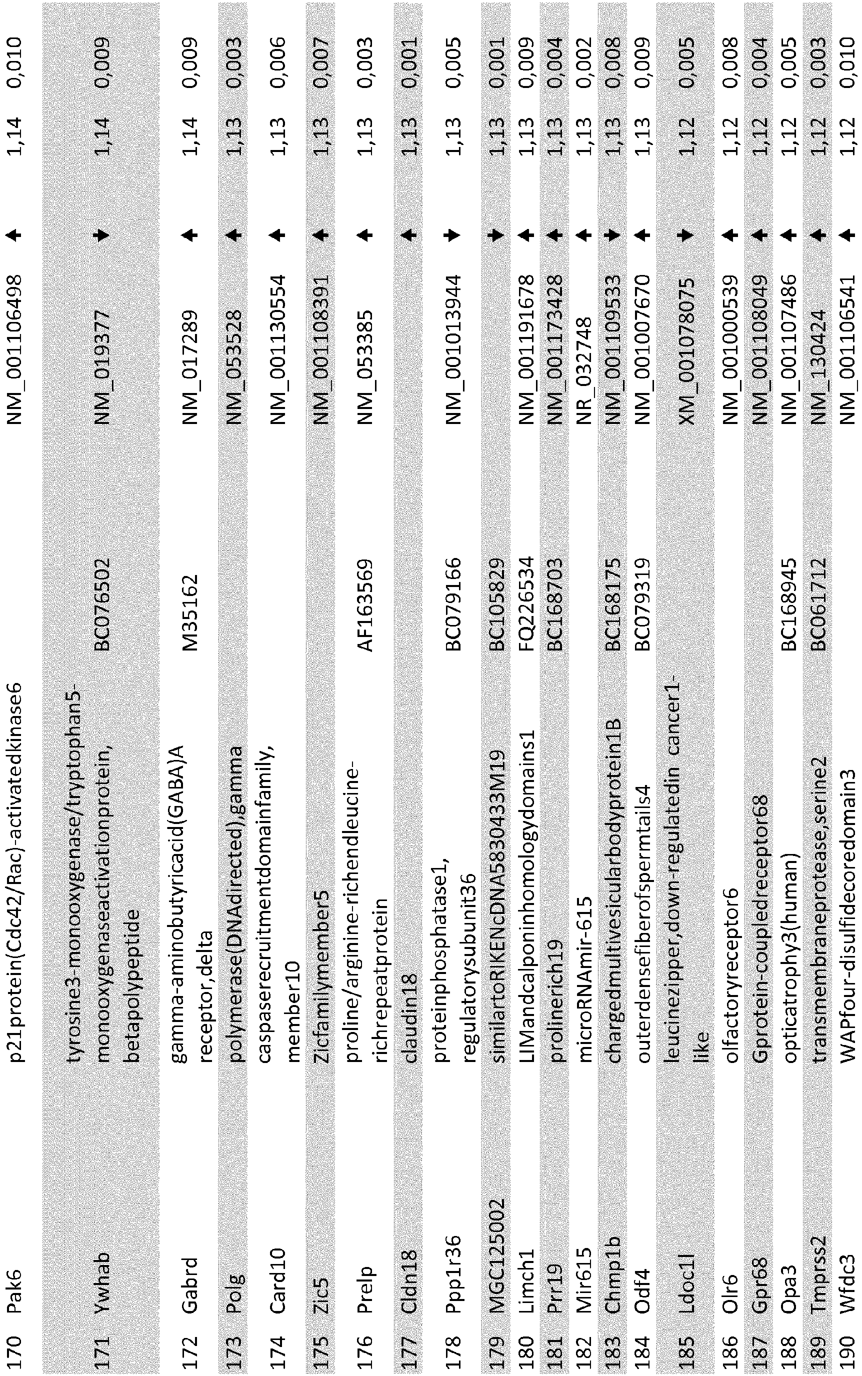

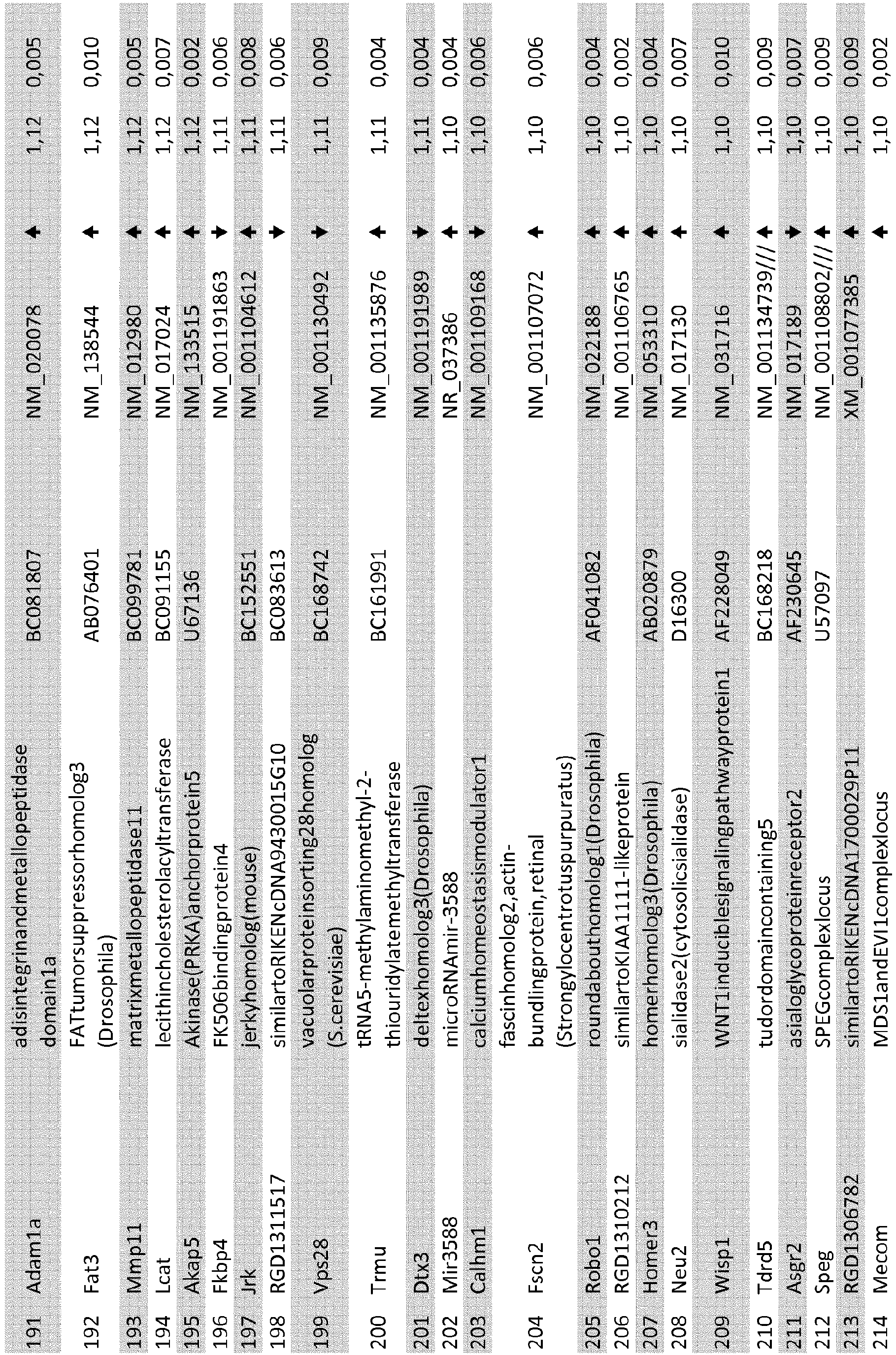

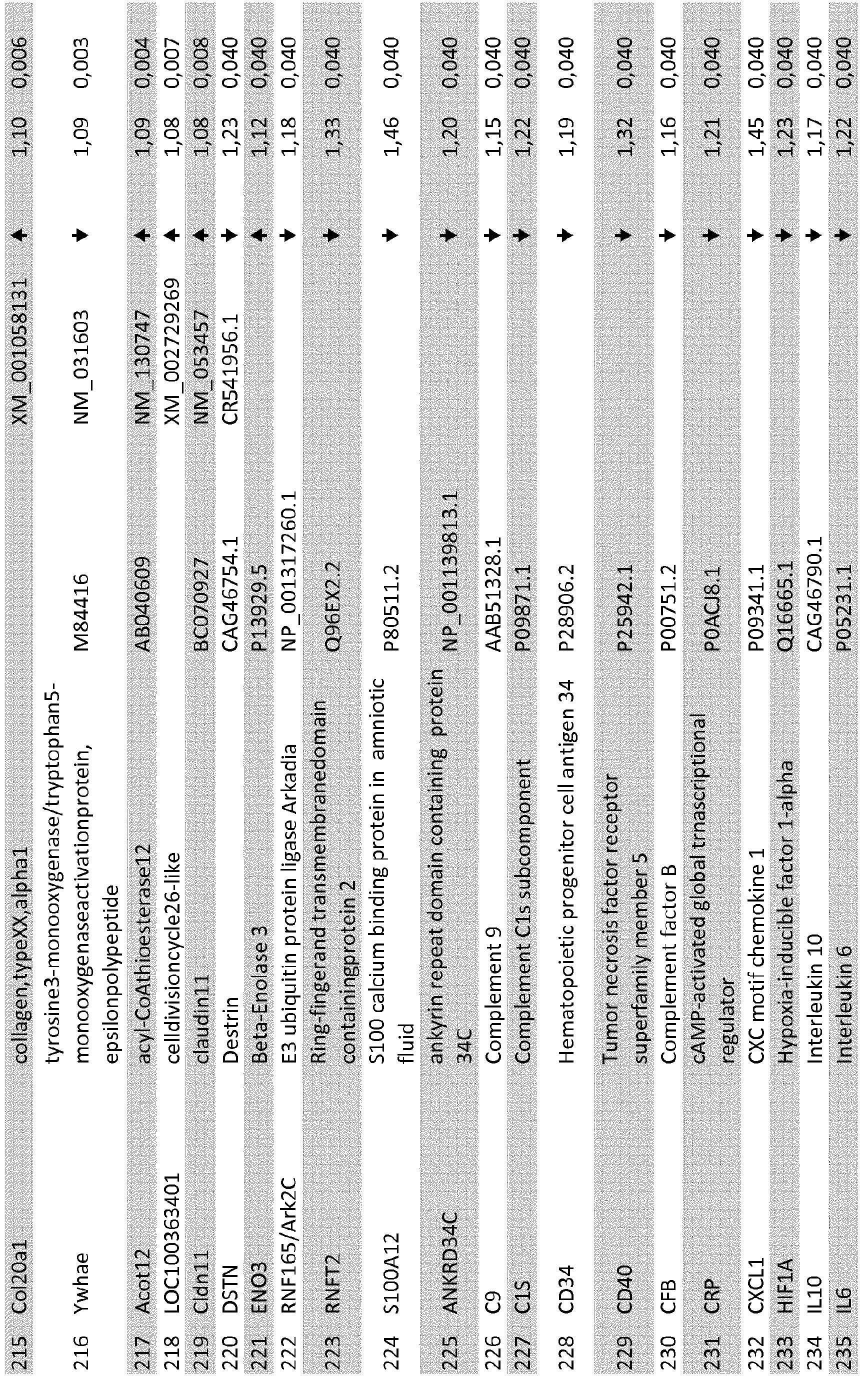

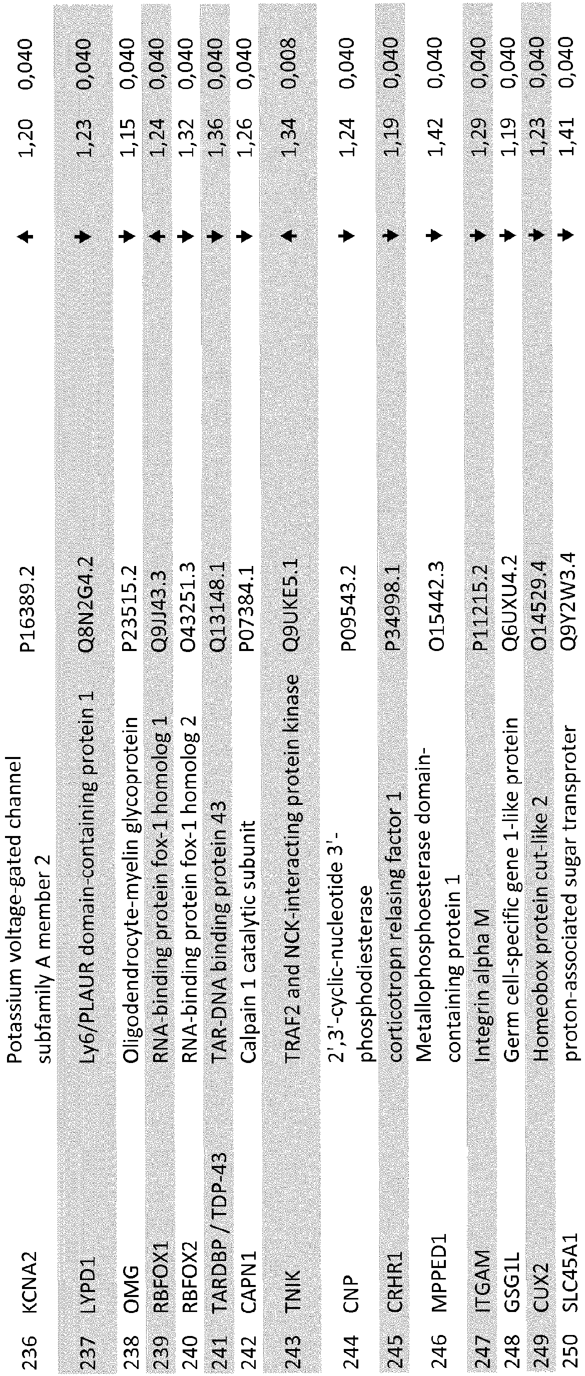

**Table 2**

| **target (rat)** | **primer forward 5'-3'** | **primer reverse 5'-3'** | **cDNA dilutior** | **add-ons** | **LM** | **TG** | | | |
|---|---|---|---|---|---|---|---|---|---|
| Ifng | GCCCTCTCTGGCTGTTACTG (SEQ ID NO: 31) concentration: 50 nM | CTGATGGCCTGGTTGTCTTT (SEQ ID NO: 32) concentration: 300 nM | 1:25 | 5% Factor Q | *n* = 7 | *n* = 3 | | | |
| Ccl4 | CTCTCTCCTCCTGCTTGTGG (SEQ ID NO: 33) concentration: 900 nM | CACAGATTTGCCTGCCTTTT (SEQ ID NO: 34) concentration: 50 nM | 1:25 | 5% Factor Q | *n* = 8 | *n* = 6 | | | |
| Il13ra1 | GAAACATGGAGGGTGCAAGT (SEQ ID NO: 35) concentration: 300 nM | CACTGCGACAAAGACTGGAA (SEQ ID NO: 36) concentration: 300 nM | 1:25 | 5% Factor Q | *n* = 7 | *n* = *5* | | | |
| Il12rb2 | AGCCTCTTAACAGCACATCCT (SEQ ID NO: 37) concentration: 300 nM | TGAAATTCATATTCTGTGAATGGTCT (SEQ ID NO: 38) concentration: 300 nM | 1:25 | no | *n* = 8 | *n* = 5 | | | |
| C3 | GAGAGCTGGTTGTGGACCAT (SEQ ID NO: 39) concentration: 50 nM | CAGTCGCAGGTCAATGAAGA (SEQ ID NO: 40) concentration: 300 nM | 1:25 | 5% Factor Q | *n* = 7 | *n* = 5 | | | |
| *Slc27a2* | GCAGGAAATACAACGCCACT (SEQ ID NO: 41) concentration: 50 nM | TCTTCCAACAGCTCCGATTT (SEQ ID NO: 42) concentration: 300 nM | 1:25 | 5% Factor Q | *n* = 8 | *n* = 5 | | | |
| Actin | GAGAGGGAAATCGTGCGTG (SEQ ID NO: 43) concentration: 300 nM | CATGGATGCCACAGGATTCC (SEQ ID NO: 44) concentration: 300 nM | dependent on target | no | | | | | |
| | | | | | batch 2 \| LMU | | | batch 1 \| Grafenberg | |

| **target (human) primer forward 5'-3'** | | **primer reverse 5'-3'** | **cDNA dilutior** | **add-ons** | **HC** | **SP** | **UR** | **CTRL** | **SCZ** |
|---|---|---|---|---|---|---|---|---|---|
| IFNG | GGCTGTAGATTCTCGAGTGCGG (SEQ ID NO: 1) concentration: 300 nM | CGCTACATCTGAATGACCTGC (SEQ ID NO: 2) concentration: 300 nM | 1:50 | no | *n* = 43 | *n* = 57 | *n* = 9 | *n* = 51 | *n* = 15 |
| CCL4 | CTGAGTTCTGCAGCCTCACC (SEQ ID NO: 3) concentration: 300 nM | CTGGGATCAGCACAGACTTGC (SEQ ID NO: 4) concentration: 300 nM | 1:10 | no | *n* = 41 | *n* = 54 | *n* = 9 | *n* = 50 | *n* = 17 |
| IL13RA1 | CCACCCGAGGGAGCCAGCTC (SEQ ID NO: 5) concentration: 50 nM | CTTCTGGGGGTGAGATGC (SEQ ID NO: 6) concentration: 50 nM | 1:10 | no | *n* = 44 | *n* = 55 | *n* = 8 | *n* = *51* | *n* = 18 |
| IL12RB2 | GACTGTGGCCTGCACCTG (SEQ ID NO: 7) concentration: 300 nM | GACAGCAGTAACCTTGGCTGTG (SEQ ID NO: 8) concentration: 300 nM | 1:10 | no | *n* = 42 | *n* = 54 | *n* = 9 | *n* = 48 | *n* = 18 |
| C3 | GCTCCAGACACAGATGACCTG (SEQ ID NO: 9) concentration: 50 nM | GCGTAGACCTTGACTGCTCCAG (SEQ ID NO: 10) concentration: 300 nM | 1:10 | no | --- | --- | --- | *n* = 45 | *n* = 17 |
| C3 | CCCTCACGGCCTTTGTTCTC (SEQ ID NO: 11) | GCCAGAGCATAGCCAGCAATG (SEQ ID NO: 12) concentration: 300 nM | 1:10 | no | *n* = 38 | *n* = 53 | *n* = 7 | --- | --- |
| SLC27A2 | CCACGACAGAGTTGGAGATAC (SEQ ID NO: 13) concentration: 300 nM | GGCCTTGCATAACTAGGTAGG (SEQ ID NO: 14) concentration: 300 nM | 1:25 | no | *n* = 43 | *n* = 57 | *n* = 8 | *n* = 49 | *n* = 18 |
| RGS1 | GAGTTCTGGCTGGCTTGTGAAG (SEQ ID NO: 15) concentration: 300 nM | GGCTGTAGATTCTCGAGTGCGG (SEQ ID NO: 16) concentration: 300 nM | 1:10 | no | *n* = 43 | *n* = 57 | *n* = 8 | *n* = 50 | *n* = 18 |
| JAK2 | GAATGTCTTGGGATGGCAGTG (SEQ ID NO: 17) concentration: 300 nM | CAGTGGCTTTGCATTGGCTG (SEQ ID NO: 18) concentration: 300 nM | 1:10 | no | *n* = 38 | *n* = 50 | *n* = 8 | --- | --- |
| CCR5 | GAGACATCCGTTCCCCTACAAG (SEQ ID NO: 19) concentration: 300 nM | GTGAGTAGAGCGGAGGCAGG (SEQ ID NO: 20) concentration: 300 nM | 1:10 | no | *n* = 38 | *n* = *51* | *n* = 8 | *n* = 31 | *n* = 13 |
| FPR2 | GTGTCCTATGAGTCTGCTGG (SEQ ID NO: 21) concentration: 300 nM | CCATGGCCATGGAGACAATG (SEQ ID NO: 22) concentration: 300 nM | 1:10 | no | *n* = 45 | *n* = 53 | *n* = 9 | --- | --- |
| NKG7 | CCCGCTTGTCTCAACCACC (SEQ ID NO: 23) concentration: 300 nM | CACAGTGAGCACCCAGGC (SEQ ID NO: 24) concentration: 300 nM | 1:10 | no | *n* = 40 | *n* = 54 | *n* = 9 | --- | --- |
| KMO | GAATGCGGGCTTTGAAGACTG (SEQ ID NO: 25) concentration: 300 nM | CGCGTGATCATCTGGGATTC (SEQ ID NO: 26) concentration: 300 nM | 1:10 | no | *n* = 44 | *n* = 53 | *n* = 9 | --- | --- |
| SERPINB1 | GACCAGAGGTAACACGGCAG (SEQ ID NO: 27) concentration: 300 nM | CACTGGCCAGGTCAGCAC (SEQ ID NO: 28) concentration: 300 nM | 1:10 | no | *n* = 39 | *n* = *51* | *n* = 8 | --- | --- |
| ARF1 | GACCACGATCCTCTACAAGC (SEQ ID NO: 29) concentration: 300 nM | TCCCACACAGTGAAGCTGATG (SEQ ID NO: 30) concentration: 300 nM | dependent on target | no | | | | | |

## Claims

1. A method for *in vitro* diagnosis of the presence of a mental disorder in a human individual or the predisposition of the human individual to the mental disorder, wherein the mental disorder is associated with a dysfunctional DISC1 protein pathway or disturbed dopamine homeostasis, the method comprising:
a) measuring in a sample of a body tissue or fluid from the individual the expression levels of at least two marker genes, each of which coding for at least one marker protein, wherein said marker genes are selected from the group consisting of human *NKG7, RGS1, CCL4, IFNG, IL12RB2, IL13RA1, KMO, FPR2, SLC27A2,* and C3;
b) comparing the measured expression levels to predetermined threshold values representing the expression levels of said marker genes in a healthy population; and
c) based on the comparison, determining whether the individual has the mental disorder or a predisposition to the mental disorder, wherein the measured expression levels are indicative to the mental disorder or disposition if the measured expression levels of said marker genes exceed, reach or fall below the predetermined threshold value.

2. The method according to claim 1, wherein a first marker gene is *RGS1* and at least one second marker gene is selected from the group consisting of human *NKG7, CCL4, IFNG, IL12RB2, IL13RA1, KMO, FPR2, SLC27A2,* and C3.

3. The method according to claim 2, wherein the first marker gene is *RGS1* and the second marker gene is *NKG7* and/or *CCL4.*

4. The method according to any of claims 1 to 3, wherein at least one additional marker gene is selected from the human equivalents of the genes listed in Table 1.

5. The method according to any of claims 1 to 4 wherein the expression levels are measured by quantitative reverse transcription Polymerase Chain Reaction or a high-affinity binding assay, and/or wherein the expression levels are measured by microarray analysis.

6. A method for determining the response to at least one pharmaceutical compound able to correct a dysfunctional DISC1 protein pathway or disturbed dopamine homeostasis, wherein the expression levels of at least two marker genes are determined and compared according to steps a) and b) of the method according to claim 1, and wherein the measured expression levels indicate that the response to the pharmaceutical compound is positive if each aberrant expression level of said marker genes is normalized or at least improved.

## Patentansprüche

1. Verfahren zur *in vitro* - Diagnose des Vorliegens einer mental-psychischen Störung bei einem menschlichen Individuum oder der Prädisposition des menschlichen Individuums für die mental-psychischen Störung, wobei die die mental-psychischen Störung mit einem funktionsgestörten DISC1-Protein-Weg oder gestörter Dopamin-Homöostase einhergeht, das Verfahren umfasst:
a) Messen der Expressionsniveaus von mindestens zwei Markergenen, von denen jedes für mindestens ein Markerprotein kodiert, in einer Probe eines Körpergewebes oder einer Körperflüssigkeit aus dem Individuum, wobei diese Markergene aus der Gruppe ausgewählt sind, die aus humanem *NKG7, RGS1, CCL4, IFNG, IL12RB2, IL13RA1, KMO, FPR2, SLC27A2* und C3 besteht;
b) Vergleichen der gemessenen Expressionsniveaus mit vorbestimmten Schwellenwerten, welche die Expressionsniveaus dieser Markergene in einer gesunden Population repräsentieren; und
c) basierend auf dem Vergleich Ermitteln, ob das Individuum die mental-psychischen Störung oder eine Prädisposition für die mental-psychischen Störung hat, wobei die gemessenen Expressionsniveaus indikativ für die mental-psychischen Störung oder die Prädisposition sind, wenn die gemessenen Expressionsniveaus der genannten Markergene den vorbestimmten Schwellenwert überschreiten, erreichen oder unterschreiten.

2. Verfahren nach Anspruch 1, wobei ein erstes Markergen *RGS1* ist und mindestens ein zweites Markergen aus der Gruppe ausgewählt ist, die aus humanem *NKG7, CCL4, IFNG, IL12RB2, IL13RA1, KMO, FPR2, SLC27A2* und C3 besteht.

3. Verfahren nach Anspruch 2, wobei das erste Markergen *RGS1* ist und das zweite Markegen *NKG7* and/or *CCL4* ist.

4. Verfahren nach einem der Ansprüche 1 bis 3, wobei mindestens ein zusätzliches Markergen aus den humanen Äquivalenten der in Tabelle 1 aufgelisteten Gene ausgewählt ist.

5. Verfahren nach einem der Ansprüche 1 bis 4, wobei die Expressionsniveaus mittels quantitativer reverser Transkriptions-Polymerasekettenreaktion oder einem hochaffinen Bindungs-Assay gemessen werden, und/oder wobei die Expressionsniveaus mittels Mikroarray-Analyse gemessen werden.

6. Verfahren zur Bestimmung der Reaktion auf mindestens einen pharmazeutischen Stoff, der geeignet ist, einen funktionsgestörten DISC1-Protein-Weg oder eine gestörte Dopamin-Homöostase zu behandeln, wobei die Expressionsniveaus von mindestens zwei Markergenen bestimmt und entsprechend der Schritte a) und b) des Verfahrens nach Anspruch 1 verglichen werden, und wobei die gemessenen Expressionsniveaus indizieren, dass die Reaktion auf den pharmazeutischen Stoff positiv ist, wenn jedes anormale Expressionsniveau der genannten Markergene normalisiert oder zumindest verbessert wurde.

## Revendications

1. Méthode de diagnostic *in vitro* de la présence d'un trouble mental chez un individu humain ou de la prédisposition de l'individu humain au trouble mental, dans laquelle le trouble mental est associé à une voie dysfonctionnelle de la protéine DISC1 ou à une homéostasie dopaminergique perturbée, la méthode comprenant :
a) la mesure dans un échantillon d'un tissu ou fluide corporel de l'individu des niveaux d'expression d'au moins deux gènes marqueurs, chacun codant pour au moins une protéine marqueur, lesdits gènes marqueurs étant sélectionnés dans le groupe constitué par *NKG7, RGS1, CCL4, IFNG, IL 12RB2, IL13RA1, KMO, FPR2, SLC27A2* et C3 humains ;
b) la comparaison des niveaux d'expression mesurés à des valeurs seuils prédéterminées représentant les niveaux d'expression desdits gènes marqueurs dans une population saine ; et
c) sur la base de la comparaison, la détermination si l'individu présente le trouble mental ou une prédisposition au trouble mental, les niveaux d'expression mesurés étant révélateurs du trouble mental ou de la disposition si les niveaux d'expression mesurés desdits gènes marqueurs dépassent, atteignent ou chutent en dessous de la valeur seuil prédéterminée.

2. Méthode selon la revendication 1, dans laquelle un premier gène marqueur est *RGS1* et au moins un deuxième gène marqueur est sélectionné dans le groupe constitué par *NKG7, CCL4, IFNG, IL12RB2, IL13RA1, KMO, FPR2, SLC27A2* et C3 humains.

3. Méthode selon la revendication 2, dans laquelle le premier gène marqueur est *RGS1* et le deuxième gène marqueur est *NKG7* et/ou *CCL4.*

4. Méthode selon l'une quelconque des revendications 1 à 3, dans laquelle au moins un gène marqueur supplémentaire est sélectionné parmi les équivalents humains des gènes énumérés dans le tableau 1.

5. Méthode selon l'une quelconque des revendications 1 à 4, dans laquelle les niveaux d'expression sont mesurés par réaction en chaîne par polymérase quantitative associée à une transcription inverse ou par un essai de liaison de haute affinité, et/ou dans laquelle les niveaux d'expression sont mesurés par analyse de microréseaux.

6. Méthode de détermination de la réponse à au moins un composé pharmaceutique capable de corriger une voie dysfonctionnelle de la protéine DISC1 ou une homéostasie dopaminergique perturbée, dans laquelle les niveaux d'expression d'au moins deux gènes marqueurs sont déterminés et comparés selon les étapes a) et b) de la méthode selon la revendication 1, et dans laquelle les niveaux d'expression mesurés indiquent que la réponse au composé pharmaceutique est positive si chaque niveau d'expression aberrant desdits gènes marqueurs est normalisé ou au moins amélioré.


### SEQUENCE LISTING

<110> Korth, Carsten
<120> Method and biomarkers for in vitro diagnosis of mental disorders
<130> 16749WO
<150> EP 16200925.2
   <151> 2016-11-28
<160> 44
<170> PatentIn version 3.5
<210> 1
   <211> 22
   <212> DNA
   <213> Artificial Sequence
<220>
   <223> Target primer forward 5'-3', IFNG (human)
<400> 1
   ggctgtagat tctcgagtgc gg 22
<210> 2
   <211> 21
   <212> DNA
   <213> Artificial Sequence
<220>
   <223> Target primer reverse 5'-3', IFNG (human)
<400> 2
   cgctacatct gaatgacctg c 21
<210> 3
   <211> 20
   <212> DNA
   <213> Artificial Sequence
<220>
   <223> Target primer forward 5'-3', CCL4 (human)
<400> 3
   ctgagttctg cagcctcacc 20
<210> 4
   <211> 21
   <212> DNA
   <213> Artificial Sequence
<220>
   <223> Target primer reverse 5'-3', CCL4 (human)
<400> 4
   ctgggatcag cacagacttg c 21
   <210> 5
   <211> 20
   <212> DNA
   <213> Artificial Sequence
<220>
   <223> Target primer forward 5'-3', IL13RA1 (human)
<400> 5
   ccacccgagg gagccagctc 20
<210> 6
   <211> 18
   <212> DNA
   <213> Artificial Sequence
<220>
   <223> Target primer reverse 5'-3', IL13RA1 (human)
<400> 6
   cttctggggg tgagatgc 18
<210> 7
   <211> 18
   <212> DNA
   <213> Artificial Sequence
<220>
   <223> Target primer forward 5'-3', IL12RB2 (human)
<400> 7
   gactgtggcc tgcacctg 18
<210> 8
   <211> 22
   <212> DNA
   <213> Artificial Sequence
<220>
   <223> Target primer reverse 5'-3', IL12RB2 (human)
<400> 8
   gacagcagta accttggctg tg 22
<210> 9
   <211> 21
   <212> DNA
   <213> Artificial Sequence
<220>
   <223> Target primer forward 5'-3', C3 (1) (human)
<400> 9
   gctccagaca cagatgacct g 21
<210> 10
   <211> 22
   <212> DNA
   <213> Artificial Sequence
<220>
   <223> Target primer reverse 5'-3', C3 (1) (human)
<400> 10
   gcgtagacct tgactgctcc ag 22
<210> 11
   <211> 20
   <212> DNA
   <213> Artificial Sequence
<220>
   <223> Target primer forward 5'-3', C3 (2) (human)
<400> 11
   ccctcacggc ctttgttctc 20
<210> 12
   <211> 21
   <212> DNA
   <213> Artificial Sequence
<220>
   <223> Target primer reverse 5'-3', C3 (2) (human)
<400> 12
   gccagagcat agccagcaat g 21
<210> 13
   <211> 21
   <212> DNA
   <213> Artificial Sequence
<220>
   <223> Target primer forward 5'-3', SLC27A2 (human)
<400> 13
   ccacgacaga gttggagata c 21
<210> 14
   <211> 21
   <212> DNA
   <213> Artificial Sequence
<220>
   <223> Target primer reverse 5'-3', SLC27A2 (human)
<400> 14
   ggccttgcat aactaggtag g 21
<210> 15
   <211> 22
   <212> DNA
   <213> Artificial Sequence
<220>
   <223> Target primer forward 5'-3', RGS1 (human)
<400> 15
   gagttctggc tggcttgtga ag 22
<210> 16
   <211> 22
   <212> DNA
   <213> Artificial Sequence
<220>
   <223> Target primer reverse 5'-3', RGS1 (human)
<400> 16
   ggctgtagat tctcgagtgc gg 22
<210> 17
   <211> 21
   <212> DNA
   <213> Artificial Sequence
<220>
   <223> Target primer forward 5'-3', JAK2 (human)
<400> 17
   gaatgtcttg ggatggcagt g 21
<210> 18
   <211> 20
   <212> DNA
   <213> Artificial Sequence
<220>
   <223> Target primer reverse 5'-3', JAK2 (human)
<400> 18
   cagtggcttt gcattggctg 20
<210> 19
   <211> 22
   <212> DNA
   <213> Artificial Sequence
<220>
   <223> Target primer forward 5'-3', CCR5 (human)
<400> 19
   gagacatccg ttcccctaca ag 22
<210> 20
   <211> 20
   <212> DNA
   <213> Artificial Sequence
<220>
   <223> Target primer reverse 5'-3', CCR5 (human)
<400> 20
   gtgagtagag cggaggcagg 20
<210> 21
   <211> 20
   <212> DNA
   <213> Artificial Sequence
<220>
   <223> Target primer forward 5'-3', FPR2 (human)
<400> 21
   gtgtcctatg agtctgctgg 20
<210> 22
   <211> 20
   <212> DNA
   <213> Artificial Sequence
<220>
   <223> Target primer reverse 5'-3', FPR2 (human)
<400> 22
   ccatggccat ggagacaatg 20
<210> 23
   <211> 19
   <212> DNA
   <213> Artificial Sequence
<220>
   <223> Target primer forward 5'-3', NKG7 (human)
<400> 23
   cccgcttgtc tcaaccacc 19
<210> 24
   <211> 18
   <212> DNA
   <213> Artificial Sequence
<220>
   <223> Target primer reverse 5'-3', NKG7 (human)
<400> 24
   cacagtgagc acccaggc 18
<210> 25
   <211> 21
   <212> DNA
   <213> Artificial Sequence
<220>
   <223> Target primer forward 5'-3', KMO (human)
<400> 25
   gaatgcgggc tttgaagact g 21
   <210> 26
   <211> 20
   <212> DNA
   <213> Artificial Sequence
<220>
   <223> Target primer reverse 5'-3', KMO (human)
<400> 26
   cgcgtgatca tctgggattc 20
<210> 27
   <211> 20
   <212> DNA
   <213> Artificial Sequence
<220>
   <223> Target primer forward 5'-3', SERPINB1 (human)
<400> 27
   gaccagaggt aacacggcag 20
<210> 28
   <211> 18
   <212> DNA
   <213> Artificial Sequence
<220>
   <223> Target primer reverse 5'-3', SERPINB1 (human)
<400> 28
   cactggccag gtcagcac 18
<210> 29
   <211> 20
   <212> DNA
   <213> Artificial Sequence
<220>
   <223> Target primer forward 5'-3', ARF1 (human)
<400> 29
   gaccacgatc ctctacaagc 20
<210> 30
   <211> 21
   <212> DNA
   <213> Artificial Sequence
<220>
   <223> Target primer reverse 5'-3', ARF1 (human)
<400> 30
   tcccacacag tgaagctgat g 21
<210> 31
   <211> 20
   <212> DNA
   <213> Artificial Sequence
<220>
   <223> Target primer forward 5'-3', Ifng (rat)
<400> 31
   gccctctctg gctgttactg 20
<210> 32
   <211> 20
   <212> DNA
   <213> Artificial Sequence
<220>
   <223> Target primer reverse 5'-3', Ifng (rat)
<400> 32
   ctgatggcct ggttgtcttt 20
<210> 33
   <211> 20
   <212> DNA
   <213> Artificial Sequence
<220>
   <223> Target primer forward 5'-3', Ccl4 (rat)
<400> 33
   ctctctcctc ctgcttgtgg 20
<210> 34
   <211> 20
   <212> DNA
   <213> Artificial Sequence
<220>
   <223> Target primer reverse 5'-3', Ccl4 (rat)
<400> 34
   cacagatttg cctgcctttt 20
<210> 35
   <211> 20
   <212> DNA
   <213> Artificial Sequence
<220>
   <223> Target primer forward 5'-3', Il13ra1 (rat)
<400> 35
   gaaacatgga gggtgcaagt 20
<210> 36
   <211> 20
   <212> DNA
   <213> Artificial Sequence
<220>
   <223> Target primer reverse 5'-3', Il13ra1 (rat)
<400> 36
   cactgcgaca aagactggaa 20
<210> 37
   <211> 21
   <212> DNA
   <213> Artificial Sequence
<220>
   <223> Target primer forward 5'-3', Il12rb2 (rat)
<400> 37
   agcctcttaa cagcacatcc t 21
<210> 38
   <211> 26
   <212> DNA
   <213> Artificial Sequence
<220>
   <223> Target primer reverse 5'-3', Il12rb2 (rat)
<400> 38
   tgaaattcat attctgtgaa tggtct 26
<210> 39
   <211> 20
   <212> DNA
   <213> Artificial Sequence
<220>
   <223> Target primer forward 5'-3', C3 (rat)
<400> 39
   gagagctggt tgtggaccat 20
<210> 40
   <211> 20
   <212> DNA
   <213> Artificial Sequence
<220>
   <223> Target primer reverse 5'-3', C3 (rat)
<400> 40
   cagtcgcagg tcaatgaaga 20
<210> 41
   <211> 20
   <212> DNA
   <213> Artificial Sequence
<220>
   <223> Target primer forward 5'-3', Slc27a2 (rat)
<400> 41
   gcaggaaata caacgccact 20
<210> 42
   <211> 20
   <212> DNA
   <213> Artificial Sequence
<220>
   <223> Target primer reverse 5'-3', Slc27a2 (rat)
<400> 42
   tcttccaaca gctccgattt 20
<210> 43
   <211> 19
   <212> DNA
   <213> Artificial Sequence
<220>
   <223> Target primer forward 5'-3', Actin (rat)
<400> 43
   gagagggaaa tcgtgcgtg 19
<210> 44
   <211> 20
   <212> DNA
   <213> Artificial Sequence
<220>
   <223> Target primer reverse 5'-3', Actin (rat)
<400> 44
   catggatgcc acaggattcc 20